# EUROPEAN PATENT SPECIFICATION

(11) **EP 4 175 968 B1**
(45) Date of publication and mention of the grant of the patent: **03.07.2024**
(21) Application number: 22751458.5
(22) Date of filing: 29.07.2022
(51) Int. Cl.: A61P 31/12, C07K 1/107, C07K 1/113

(54) **METHODS AND COMPOSITIONS FOR PROTEIN SYNTHESIS AND SECRETION**
VERFAHREN UND ZUSAMMENSETZUNGEN ZUR PROTEINSYNTHESE UND -SEKRETATION
PROCEDES ET COMPOSITIONS POUR LA SYNTHESE ET LA SECRETION DE PROTEINES

(30) Priority: 30.07.2021 US 202163227820 P; 29.10.2021 US 202163273858 P
(43) Date of publication of application: 10.05.2023
(73) Proprietor: Helaina, Inc., New York, NY 10010 (US)
(72) Inventor: BOTERO BESADA-LOMBANA, Pamela, New York, New York 10010 (US); KATZ, Laura, New York, New York 10010 (US)
(74) Representative: Bohmann, Armin K.
(86) International application number: PCT/IB2022/057092
(87) International publication number: WO 2023/007468

(56) References cited:
- WO-A1-2012/152823
- WO-A1-2014/066479
- CHAHAL SABREEN ET AL: "Structural characterization of the [alpha]-mating factor prepro-peptide for secretion of recombinant proteins in Pichia pastoris", GENE, vol. 598, 1 January 2017 (2017-01-01), pages 50-62, XP55972786, NL ISSN: 0378-1119, DOI: 10.1016/j.gene.2016.10.040
- HEISS SILVIA ET AL: "Multistep processing of the secretion leader of the extracellular protein Epx1 in Pichia pastoris and implications for protein localization", MICROBIOLOGY, vol. 161, no. 7, 1 July 2015 (2015-07-01), pages 1356-1368, XP55972469, Reading ISSN: 1350-0872, DOI: 10.1099/mic.0.000105 Retrieved from the Internet: URL:https://www.microbiologyresearch.org/d ocserver/fulltext/micro/161/7/1356_mic0001 05.pdf?expires=1666100366&id=id&accname=gu est&checksum=F3399BFD1D30C707BCB8895C2B264 CBC> cited in the application
- DE SCHUTTER KRISTOF ET AL: "Genome sequence of the recombinant protein production host Pichia pastoris", NATURE BIOTECHNOLOGY, NATURE PUBLISHING GROUP US, NEW YORK, vol. 27, no. 6, 1 June 2009 (2009-06-01), page 561, XP002544776, ISSN: 1087-0156, DOI: 10.1038/NBT.1544 [retrieved on 2009-05-24]
- NANDI S ET AL: "Expression of human lactoferrin in transgenic rice grains for the application in infant formula", PLANT SCIENCE, ELSEVIER IRELAND LTD, IE, vol. 163, no. 4, 1 October 2002 (2002-10-01), pages 713-722, XP002310363, ISSN: 0168-9452, DOI: 10.1016/S0168-9452(02)00165-6
- DURAN: "THE USE OF LACTOFERRIN IN FOOD INDUSTRY", ACADEMIC JOURNAL OF SCIENCE, 1 December 2017 (2017-12-01), XP55972489, cited in the application

## Description

### BACKGROUND

### I. Technical Field

Aspects of this invention relate to at least the fields of microbiology, genetics, and biotechnology. More specifically the present invention relates to an isolated nucleic acid encoding a polypeptide, a vector comprising the nucleic acid, an engineered *Pichia pastoris* cell comprising the nucleic acid or the vector, and a method for producing a secreted protein.

### II. Background

Yeast is a desirable host for production of recombinant proteins due to its rapid growth, its ability to reach high cell densities, to grow on defined minimal media, achieve high protein yields and conduct eukaryotic post-translational modifications. The most relevant yeast for protein production is *Pichia pastoris* (*Komagataella pastoris, Komagataella phaffii*) due to the wide availability of genomic information and molecular tools for genomic manipulation. These have enabled the use of *Pichia pastoris* for production of GRAS ingredients based on the FDA criteria.

For diverse biotechnological applications it is often preferred to produce proteins that are secreted to the growth medium to ease recovery. *Pichia pastoris* is capable of secreting active recombinant proteins, while maintaining low-level secretion of endogenous proteins.

In eukaryotes, secreted proteins are first targeted from the cytoplasm to the lumen endoplasmic reticulum (ER) via translocation. Translocation into the ER can take place either post-translationally (i.e., once the polypeptide chain has been synthesized) or co-translationally (i.e., during mRNA translation into its amino acid sequence). Post-translational translocation requires chaperones that maintain the polypeptide chain in a loose conformation in the cytosol as well as the action of the ER-resident chaperone Kar2, which acts as a molecular ratchet. Consequently, this process can be hindered by partially folded domains and/or cytosolic aggregation. Therefore, for biotechnological applications it is desirable to promote co-translational translocation. Once in the ER, proteins are glycosylated, their disulfide bonds are isomerized, and they fold to their native state. Proteins that are successfully folded then transit to the Golgi complex, where further glycosylation takes place before being packed into secretory granules that fuse to the cell membrane, releasing the protein to the extracellular milieu.

Targeting of the proteins to the secretory pathway is mediated by secretion peptides. The most widely used in *Pichia pastoris* is the leader peptide of the mating factor alpha of *S. cerevisiae.* It is comprised of two distinct regions: ii) the first 19 amino acid pre-region that promotes post-translational translocation and is cleaved upon ER entry ii) a 70 amino acid pro-segment that serves as an ER-to-Golgi export signal and it is cleaved in the Golgi Apparatus at the dibasic amino acid cleavage site KR.

There exists a need for synthetic secretion signal peptides leading to higher extracellular production of proteins.

### SUMMARY

The problem underlying the present invention is solved by the subject matter of the attached independent claims; preferred embodiments may be taken from the attached dependent claims.

More specifically, the problem underlying the present invention is solved in a first aspect by an isolated nucleic acid encoding a polypeptide comprising a sequence having at least 90% sequence identity to SEQ ID NO: 1, 2, 3, or 4.

In an embodiment of the first aspect, the polypeptide sequence comprises SEQ ID NO: 1, 2, 3, or 4.

In an embodiment of the first aspect, the polypeptide further comprises a sequence of a mammalian protein.

In an embodiment of the first aspect, the mammalian protein is a human milk protein.

In an embodiment of the first aspect, the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin, optionally wherein the human milk protein is human lactoferrin.

In an embodiment of the first aspect, the polypeptide sequence comprises SEQ ID NO:3.

In an embodiment of the first aspect, the isolated nucleic acid comprises a nucleic acid sequence having at least 80% identity to SEQ ID NO:41, 42, 43, or 44.

In an embodiment of the first aspect, the nucleic acid sequence comprises SEQ ID NO:41, 42, 43, or 44.

In an embodiment of the first aspect, the polypeptide comprises SEQ ID NO:5, 6, 7, or 8.

In an embodiment of the first aspect, the isolated nucleic acid comprises a nucleic acid sequence having at least 80% identity to SEQ ID NO:46, 47, 48, or 49.

In an embodiment of the first aspect, the nucleic acid sequence comprises SEQ ID NO:46, 47, 48, or 49.

More specifically, the problem underlying the present invention is solved in a second aspect by a vector comprising the nucleic acid according to the first aspect, including any embodiment thereof.

More specifically, the problem underlying the present invention is solved in a third aspect by an engineered *Pichia pastoris* cell comprising the nucleic acid according to the first aspect, including any embodiment thereof, or the vector according to the second aspect, including any embodiment thereof.

In an embodiment of the third aspect, the nucleic acid encodes a mammalian polypeptide.

In an embodiment of the third aspect, the sequence comprises SEQ ID NO:1, 2, 3, or 4, optionally wherein the sequence comprises SEQ ID NO:3.

In an embodiment of the third aspect, the polypeptide is a mammalian protein, optionally wherein the mammalian protein is a human milk protein.

In an embodiment of the third aspect, the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin, optionally wherein the human milk protein is human lactoferrin.

In an embodiment of the third aspect, the Pichia pastoris cell is Pichia pastoris, Komagataella pastoris or Komagataella phaffii.

More specifically, the problem underlying the present invention is solved in a fourth aspect by a method for producing a secreted protein, the method comprising growing the *Pichia pastoris* cell according to the third aspect, including any embodiment thereof, under conditions sufficient to secrete the polypeptide from the cell, optionally comprising collecting the secreted protein.

In an embodiment of the fourth aspect, the secreted protein is a human milk protein, optionally wherein the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin.

In an embodiment of the fourth aspect, the human milk protein comprises one or more human-like N-glycans.

In an embodiment of the fourth aspect, the method further comprises generating a mixture comprising the human milk protein and one or more components of an infant formula.

In an embodiment of the fourth aspect, the Pichia pastoris cell is Pichia pastoris, Komagataella pastoris or Komagataella phaffii.

The present invention as defined in the claims addresses certain needs by providing novel secretion signal peptides effective in improving extracellular production of proteins, including mammalian proteins such as, for example, human milk proteins. Certain aspects of the present invention as defined in the claims are based, at least in part, on the development of signal peptides generated from the in-frame fusion of 1) pre-secretion peptides of *P. pastoris* from either i) the alpha subunit of the oligosaccharyltransferase complex of the ER lumen (Ost1) or ii) the GPI-anchored protein Pst1 with 2) the pro-region of either i) the *S. cerevisiae* mating factor or ii) pro-region of *P. pastoris* Epx1. Accordingly, described herein an subject to the present invention as defined in the claims are isolated nucleic acids encoding such secretion signal peptides, in some cases linked to a recombinant protein such as a human milk protein, as well as cells comprising such nucleic acids and methods for producing and collecting recombinant proteins from such cells.

The present invention as defined in the claims is related to an isolated nucleic acid encoding a polypeptide comprising a sequence having at least 90% sequence identity to SEQ ID NO: 1, 2, 3, or 4. In some embodiments, the sequence comprises SEQ ID NO:1, 2, 3, or 4. In some embodiments of the present invention as defined in the claims, the polypeptide further comprises a sequence of a mammalian protein. In some embodiments of the present invention as defined in the claims, the mammalian protein is a human milk protein. In some embodiments, the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin. In some embodiments, the human milk protein is human lactoferrin.

In some embodiments of the present invention as defined in the claims, the sequence has at least 90%, 91%, 92%, 93%, 94%, 95%, 96%, 97%, 98%, or 99% sequence identity to SEQ ID NO: 1. In some embodiments of the present invention as defined in the claims, the sequence comprises SEQ ID NO:1. In some embodiments of the present invention as defined in the claims, the isolated nucleic acid comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:41. In some embodiments of the present invention as defined in the claims, the nucleic acid sequence comprises SEQ ID NO:41. In some embodiments of the present invention as defined in the claims, the polypeptide comprises SEQ ID NO:5. In some embodiments of the present invention as defined in the claims, the isolated nucleic acid comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:46. In some embodiments of the present invention as defined in the claims, the nucleic acid sequence comprises SEQ ID NO:46.

In some embodiments of the present invention as defined in the claims, the sequence has at least 90%, 91%, 92%, 93%, 94%, 95%, 96%, 97%, 98%, or 99% sequence identity to SEQ ID NO:2. In some embodiments of the present invention as defined in the claims, the sequence comprises SEQ ID NO:2. In some embodiments of the present invention as defined in the claims, the isolated nucleic acid comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:42. In some embodiments of the present invention as defined in the claims, the nucleic acid sequence comprises SEQ ID NO:42. In some embodiments of the present invention as defined in the claims, the polypeptide comprises SEQ ID NO:6. In some embodiments of the present invention as defined in the claims, the isolated nucleic acid comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:47. In some embodiments of the present invention as defined in the claims, the nucleic acid sequence comprises SEQ ID NO:47.

In some embodiments of the present invention as defined in the claims, the sequence has at least 90%, 91%, 92%, 93%, 94%, 95%, 96%, 97%, 98%, or 99% sequence identity to SEQ ID NO:3. In some embodiments of the present invention as defined in the claims, the sequence comprises SEQ ID NO:3. In some embodiments of the present invention as defined in the claims, the isolated nucleic acid comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:43. In some embodiments of the present invention as defined in the claims, the nucleic acid sequence comprises SEQ ID NO:43. In some embodiments of the present invention as defined in the claims, the polypeptide comprises SEQ ID NO:7. In some embodiments of the present invention as defined in the claims, the isolated nucleic acid comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:48. In some embodiments of the present invention as defined in the claims, the nucleic acid sequence comprises SEQ ID NO:48.

In some embodiments of the present invention as defined in the claims, the sequence has at least 90%, 91%, 92%, 93%, 94%, 95%, 96%, 97%, 98%, or 99% sequence identity to SEQ ID NO:4. In some embodiments of the present invention as defined in the claims, the sequence comprises SEQ ID NO:4. In some embodiments of the present invention as defined in the claims, the isolated nucleic acid comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:44. In some embodiments of the present invention as defined in the claims, the nucleic acid sequence comprises SEQ ID NO:44. In some embodiments of the present invention as defined in the claims, the polypeptide comprises SEQ ID NO:8. In some embodiments of the present invention as defined in the claims, the isolated nucleic acid comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:49. In some embodiments of the present invention as defined in the claims, the nucleic acid sequence comprises SEQ ID NO:49.

The present invention as defined in the claims is also related to a vector comprising a nucleic acid (e.g., an isolated nucleic acid or sequence or portion thereof) of the present invention as defined in the claims.

The present invention as defined in the claims is additionally related to an engineered eukaryotic cell, namely *Pichia pastoris*, comprising a nucleic acid of the present invention as defined in the claims. In some examples, the eukaryotic cell is a fungal cell. In some examples, the fungal cell is a *Arxula*, *Aspegillus*, *Aurantiochytrium*, *Candida*, *Claviceps*, *Cryptococcus, Cunninghamella, Geotrichum, Hansenula, Kluyveromyces, Kodamaea, Komagataella, Leucosporidiella, Lipomyces*, *Mortierella, Ogataea, Pichia, Prototheca, Rhizopus, Rhodosporidium, Rhodotorula, Saccharomyces, Schizosaccharomyces, Tremella, Trichosporon*, *Wickerhamomyces*, or *Yarrowia* cell. In some examples, the cell is a yeast cell. In some embodiments of the present invention as defined in the claims, the cell is a *Komagataella* cell. In some embodiments of the present invention as defined in the claims, the yeast cell is a *Komagataella phaffii* or a *Komagataella pastoris.* Also disclosed herein as eukaryotic cell is a *Komagataella pseudopastoris* cell. In some embodiments of the present invention as defined in the claims, the nucleic acid is integrated into the genome of the cell. In some embodiment of the present invention as defined in the claims, the nucleic acid is not integrated into the genome of the cell.

Furthermore, the present invention as defined in the claims is related to a method for producing a secreted protein, the method comprising growing an engineered eukaryotic cell of the present invention as defined in the claims under conditions sufficient to secrete the polypeptide from the cell. In some embodiments of the present invention as defined in the claims, the method further comprises collecting the secreted protein. In some embodiments of the present invention as defined in the claims, the secreted protein is a human milk protein. In some embodiments of the present invention as defined in the claims, the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin. In some embodiments of the present invention as defined in the claims, the human milk protein is human lactoferrin. In some embodiments of the present invention as defined in the claims, the human milk protein comprises one or more human-like N-glycans. In some embodiments of the present invention as defined in the claims, the method further comprises generating a mixture comprising the human milk protein and one or more components of an infant formula.

The engineered yeast cell of the present invention as defined in the claims comprises a nucleic acid encoding a polypeptide comprising a sequence having at least 90% sequence identity to SEQ ID NO:1, 2, 3, or 4. In some embodiments of the present invention as defined in the claims, the sequence comprises SEQ ID NO:1, 2, 3, or 4. In some embodiments of the present invention as defined in the claims, the sequence comprises SEQ ID NO: 1. In some embodiments of the present invention as defined in the claims, the sequence comprises SEQ ID NO:2. In some embodiments of the present invention as defined in the claims, the sequence comprises SEQ ID NO:3. In some embodiments of the present invention as defined in the claims, the sequence comprises SEQ ID NO:4. In some embodiments of the present invention as defined in the claims, the polypeptide further comprises a sequence of a mammalian protein. In some embodiments of the present invention as defined in the claims, the mammalian protein is a human milk protein. In some embodiments, the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin. In some embodiments, the human milk protein is human lactoferrin.

In an embodiment of the present invention as defined in the claims, the engineered yeast cell comprises: (a) a first nucleic acid encoding a polypeptide comprising: (i) a sequence having at least 90% sequence identity to SEQ ID NO: 1, 2, 3, or 4 and (ii) a sequence of a human milk protein; and (b) a second nucleic acid encoding an alpha-1,2-mannosidase (Man-I) protein, wherein the cell does not express a functional OCH1 protein. In some embodiments, the sequence of (i) comprises SEQ ID NO:1, 2, 3, or 4. In some embodiments of the present invention as defined in the claims, the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin. In some embodiments of the present invention as defined in the claims, the human milk protein is human lactoferrin. In some embodiments of the present invention as defined in the claims, the human milk protein is human α-lactalbumin. In some embodiments of the present invention as defined in the claims, the Man-I protein is fused to a HDEL C-terminal tag. In some embodiments of the present invention as defined in the claims, the cell further comprises a third nucleic acid encoding one or more of: (a) a N-acetylglucosaminyltransferase I (GnT-I) protein; (b) an α-1,3/6-Mannosidase (Man-II) protein; (c) a β-1,2-acetylglucosaminyltransferase (GnT-II) protein; and (d) a β-1,4-galactosyltransferase (GalT) protein. In some embodiments of the present invention as defined in the claims, the yeast cell is a *Komagataella phaffii* or *Komagataella pastoris* cell. In some embodiments of the present invention as defined in the claims, the nucleic acid is integrated into the genome of the cell. In some embodiments of the present invention as defined in the claims, the nucleic acid is not integrated into the genome of the cell.

It is contemplated that any embodiment of the present invention as defined in the claims discussed in this specification can be implemented with respect to any aspect of the present invention as defined in the claims.

### BRIEF DESCRIPTION OF THE DRAWINGS

The following drawings form part of the present specification and are included to further demonstrate certain aspects of the present invention as defined in the claims. This may be better understood by reference to one or more of these drawings in combination with the detailed description of specific embodiments of the present invention as defined in the claims and presented herein.

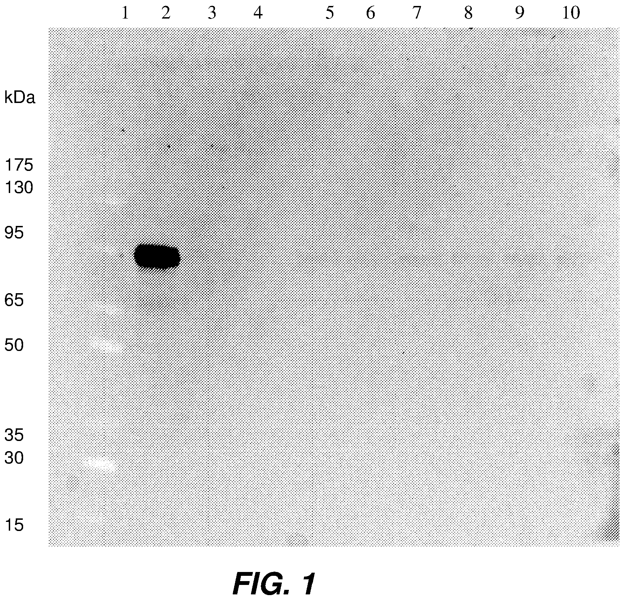

**FIG. 1** is an image of a Western Blot of supernatants. Lane 1 is loaded with a protein standard, Genscript, M00624 (ThermoFisher Scientific, Waltham, MA, USA). Lane 2 is loaded with lactoferrin from Human Milk, Sigma Aldrich, SRP6519 (Sigma Aldrich, St. Louis, MO, USA). Lane 3 is loaded with a control (Saccharomyces cerevisiae pre-pro-MFα). Lane 4 is loaded with the negative control, a supernatant of untransformed yeast cells. Lanes 5-6 are loaded with supernatant from SP2-lactoferrin transformed yeast cells. Lanes 7-8 are loaded with supernatant from SP3-lactoferrin transformed yeast cells. Lanes 9-10 are loaded with SP1-lactoferrin transformed yeast cells.

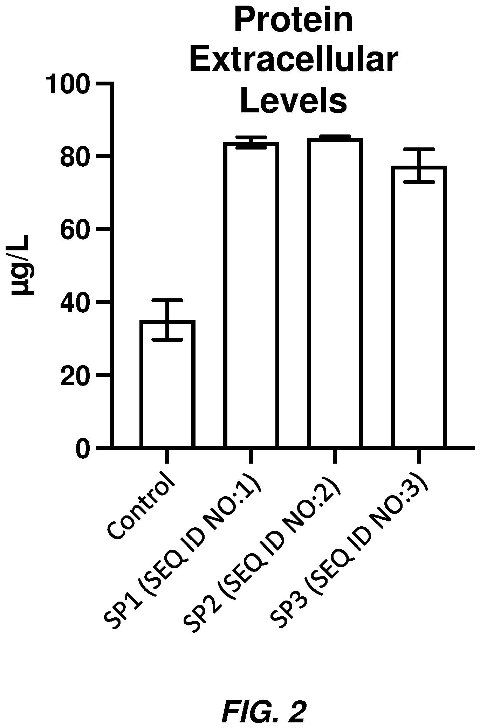

**FIG. 2** is a bar graph showing protein expression levels. Quantification of extracellular protein was performed via ELISA.

### DETAILED DESCRIPTION

The present invention as defined in the claims is related to novel synthetic secretion signal peptides. The present invention as defined in the claims is also related to a cell engineered to express one or more exogenous proteins (e.g., human milk proteins) comprising such signal peptides. In accordance with the present invention and as disclosed herein, the in-frame fusion of "pre-region" sequences from *P. pastoris* Ost1 or Pst1 and "pro-region" sequences from *S. cerevisiae* mating factor α or *P. pastoris* Epx1 can facilitate increased extracellular protein production compared with previously used signal peptides. The signal peptides include, for example, peptides comprising SEQ ID NOs:1, 2, 3, or 4, as well as peptides comprising 1, 2, 3, 4, or 5 amino acid substitutions (or more) relative to SEQ ID NO:1, 2, 3, or 4. As described herein, in-frame fusion of these hybrid signal peptides to the N-terminus of mammalian proteins (e.g., human milk proteins such as lactoferrin or α-lactalbumin) promotes highly efficient protein secretion.

### I. Definitions

The term "biologically-active portion" refers to an amino acid sequence that is less than a full-length amino acid sequence, but exhibits at least one activity of the full length sequence. For example, a biologically-active portion of an enzyme may refer to one or more domains of an enzyme having the catalytic activity of the enzyme (i.e., may be a catalytic domain). In some embodiments, a biologically-active portion of an enzyme is a portion of the enzyme comprising a catalytic domain of the enzyme. Biologically-active portions of a protein include peptides or polypeptides comprising amino acid sequences sufficiently identical to or derived from the amino acid sequence of the protein, which include fewer amino acids than the full length protein, and exhibit at least one activity (e.g., enzymatic activity, functional activity, etc.) of the protein.

The term "exogenous" refers to anything that is introduced into a cell or has been introduced into a cell. An "exogenous nucleic acid" is a nucleic acid that enters or has entered a cell through the cell membrane. An "exogenous nucleic acid sequence" is a nucleic acid sequence of an exogenous nucleic acid. An exogenous nucleic acid may contain a nucleotide sequence that exists in the native genome of a cell and/or nucleotide sequences that did not previously exist in the cell's genome. Exogenous nucleic acids include exogenous genes. An "exogenous gene" is a nucleic acid that codes for the expression of an RNA and/or protein that has been introduced into a cell (e.g., by transformation/transfection), and is also referred to as a "transgene." A cell comprising an exogenous nucleic acid may be referred to as a recombinant cell, into which additional exogenous gene(s) may be introduced. The exogenous gene may be from the same or different species relative to the cell being transformed. Thus, an exogenous gene can include a native gene that occupies a different location in the genome of the cell or is under different control, relative to the endogenous copy of the gene. An exogenous gene may be present in more than one copy in the cell. An exogenous gene may be maintained in a cell as an insertion into the genome (nuclear, mitochondrial, or plastid) or as an episomal molecule.

"In operable linkage" (or "operably linked") refers to a functional linkage between two nucleic acid sequences, such a control sequence (typically a promoter) and the linked sequence (typically a sequence that encodes a protein, also called a coding sequence). A promoter is in operable linkage with a gene if it can mediate transcription of the gene.

The term "native" refers to the composition of a cell or parent cell prior to a transformation event. A "native gene" (also "endogenous gene") refers to a nucleotide sequence that encodes a protein that has not been introduced into a cell by a transformation event. A "native protein" (also "endogenous protein") refers to an amino acid sequence that is encoded by a native gene.

"Recombinant" refers to a cell, nucleic acid, protein, or vector, which has been modified due to introduction of an exogenous nucleic acid or alteration of a native nucleic acid. Resulting cells, nucleic acids, proteins or vectors are considered recombinant, as are progeny, offspring, duplications or replications of these are also considered recombinant. Thus, e.g., recombinant cells can express genes that are not found within the native (non-recombinant) form of the cell or express native genes differently than those same genes are expressed by a non-recombinant cell. Recombinant cells can, without limitation, include recombinant nucleic acids that encode for a gene product or for suppression elements such as mutations, knockouts, antisense, interfering RNA (RNAi), or dsRNA that reduce the levels of active gene product in a cell. A "recombinant nucleic acid" is derived from nucleic acid originally formed in vitro, in general, by the manipulation of nucleic acid, e.g., using polymerases, ligases, exonucleases, and endonucleases, or otherwise is in a form not normally found in nature. Once a recombinant nucleic acid is made and introduced into a host cell or organism, it may replicate using the in vivo cellular machinery of the host cell; however, such nucleic acids, once produced recombinantly, although subsequently replicated intracellularly, are still considered recombinant for purposes of this disclosure. Additionally, a recombinant nucleic acid refers to nucleotide sequences that comprise an endogenous nucleotide sequence and an exogenous nucleotide sequence; thus, an endogenous gene that has undergone recombination with an exogenous promoter is a recombinant nucleic acid. A "recombinant protein" is a protein made using recombinant techniques, i.e., through the expression of a recombinant nucleic acid.

"Transformation" refers to the transfer of a nucleic acid into a host organism or the genome of a host organism. Host organisms (and their progeny) containing the transformed nucleic acid fragments are referred to as "recombinant", "transgenic" or "transformed" organisms. Thus, isolated polynucleotides can be incorporated into recombinant constructs, typically DNA constructs, capable of introduction into and replication in a host cell. Such a construct can be a vector that includes a replication system and sequences that are capable of transcription and translation of a polypeptide-encoding sequence in a given host cell. Typically, expression vectors include, for example, one or more cloned genes under the transcriptional control of 5' and 3' regulatory sequences and a selectable marker. Such vectors also can contain a promoter regulatory region (e.g., a regulatory region controlling inducible or constitutive, environmentally- or developmentally-regulated, or location-specific expression), a transcription initiation start site, a ribosome binding site, a transcription termination site, and/or a polyadenylation signal. Alternatively, a cell may be transformed with a single genetic element, such as a promoter, which may result in genetically stable inheritance upon integrating into the host organism's genome, such as by homologous recombination.

The term "transformed cell" refers to a cell that has undergone a transformation. Thus, a transformed cell comprises the parent's genome and an inheritable genetic modification. Embodiments include progeny and offspring of such transformed cells.

The term "vector" refers to the means by which a nucleic acid can be propagated and/or transferred between organisms, cells, or cellular components. Vectors include plasmids, linear DNA fragments, viruses, bacteriophage, pro-viruses, phagemids, transposons, and artificial chromosomes, and the like, that may or may not be able to replicate autonomously or integrate into a chromosome of a host cell.

"Individual," "subject," and "patient" are used interchangeably and can refer to a human or non-human.

Throughout this application, the term "about" is used to indicate that a value includes the inherent variation of error for the measurement or quantitation method.

The use of the word "a" or "an" when used in conjunction with the term "comprising" may mean "one," but it is also consistent with the meaning of "one or more," "at least one," and "one or more than one."

The phrase "and/or" means "and" or "or". To illustrate, A, B, and/or C includes: A alone, B alone, C alone, a combination of A and B, a combination of A and C, a combination ofB and C, or a combination of A, B, and C. In other words, "and/or" operates as an inclusive or.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The compositions and methods for their use can "comprise," "consist essentially of," or "consist of" any of the ingredients or steps disclosed throughout the specification. Compositions and methods "consisting essentially of" any of the ingredients or steps disclosed limits the scope of the claim to the specified materials or steps which do not materially affect the basic and novel characteristic of the claimed embodiment.

### II. Proteins and Nucleic Acids

As used herein, a "protein" or "polypeptide" refers to a molecule comprising at least five amino acid residues. As used herein, the term "wild-type" refers to the endogenous version of a molecule that occurs naturally in an organism. In some embodiments of the present invention as defined in the claims, wild-type versions of a protein or polypeptide are employed, however, in many embodiments of the present invention as defined in the claims , a modified protein or polypeptide is employed. The terms described above may be used interchangeably. A "modified protein" or "modified polypeptide" or a "variant" refers to a protein or polypeptide whose chemical structure, particularly its amino acid sequence, is altered with respect to the wild-type protein or polypeptide. In some embodiments of the present invention as defined in the claims, a modified/variant protein or polypeptide has at least one modified activity or function (recognizing that proteins or polypeptides may have multiple activities or functions). It is specifically contemplated that a modified/variant protein or polypeptide may be altered with respect to one activity or function yet retain a wild-type activity or function in other respects.

Where a protein is specifically mentioned herein, it is in general a reference to a native (wild-type) or recombinant (modified) protein or, optionally, a protein in which any signal sequence has been removed. The protein may be isolated directly from the organism of which it is native, produced by recombinant DNA/exogenous expression methods, or produced by solid-phase peptide synthesis (SPPS) or other in vitro methods. In particular embodiments of the present invention as defined in the claims, there are isolated nucleic acid segments and recombinant vectors incorporating nucleic acid sequences that encode a polypeptide. The term "recombinant" may be used in conjunction with a polypeptide or the name of a specific polypeptide, and this generally refers to a polypeptide produced from a nucleic acid molecule that has been manipulated in vitro or that is a replication product of such a molecule.

In certain embodiments of the present invention as defined in the claims, the size of a protein or polypeptide (wild-type or modified) may comprise, but is not limited to, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000, 1100, 1200, 1300, 1400, 1500, 1750, 2000, 2250, 2500 amino acid residues or greater, and any range derivable therein, or derivative of a corresponding amino sequence described or referenced herein. It is contemplated that polypeptides may be mutated by truncation, rendering them shorter than their corresponding wild-type form, also, they might be altered by fusing or conjugating a heterologous protein or polypeptide sequence with a particular function (e.g., for targeting or localization, for enhanced immunogenicity, for purification purposes, etc.). As used herein, the term "domain" refers to any distinct functional or structural unit of a protein or polypeptide, and generally refers to a sequence of amino acids with a structure or function recognizable by one skilled in the art.

The term "polynucleotide" refers to a nucleic acid molecule that either is recombinant or has been isolated from total genomic nucleic acid. Included within the term "polynucleotide" are oligonucleotides (nucleic acids 100 residues or less in length), recombinant vectors, including, for example, plasmids, cosmids, phage, viruses, and the like. Polynucleotides include, in certain aspects, regulatory sequences, isolated substantially away from their naturally occurring genes or protein encoding sequences. Polynucleotides may be single- stranded (coding or antisense) or double- stranded, and may be RNA, DNA (genomic, cDNA or synthetic), analogs thereof, or a combination thereof. Additional coding or noncoding sequences may, but need not, be present within a polynucleotide.

In this respect, the term "gene," "polynucleotide," or "nucleic acid" is used to refer to a nucleic acid that encodes a protein, polypeptide, or peptide (including any sequences required for proper transcription, post-translational modification, or localization). As will be understood by those in the art, this term encompasses genomic sequences, expression cassettes, cDNA sequences, and smaller engineered nucleic acid segments that express, or may be adapted to express, proteins, polypeptides, domains, peptides, fusion proteins, and mutants. A nucleic acid encoding all or part of a polypeptide may contain a contiguous nucleic acid sequence encoding all or a portion of such a polypeptide. It also is contemplated that a particular polypeptide may be encoded by nucleic acids containing variations having slightly different nucleic acid sequences but, nonetheless, encode the same or substantially similar protein.

In certain embodiments of the present invention as defined in the claims, there are polynucleotide variants having substantial identity to the sequences disclosed herein; those comprising at least 70%, 75%, 80%, 85%, 90%, 95%, 96%, 97%, 98%, or 99% or higher sequence identity, including all values and ranges there between, compared to a polynucleotide sequence provided herein using the methods described herein (e.g., BLAST analysis using standard parameters). In certain embodiments of the present invention as defined in the claims, the isolated polynucleotide will comprise a nucleotide sequence encoding a polypeptide that has at least 90%, and in some cases 95% and above, identity to an amino acid sequence described herein, over the entire length of the sequence; or a nucleotide sequence complementary to said isolated polynucleotide.

The nucleic acid segments, regardless of the length of the coding sequence itself, may be combined with other nucleic acid sequences, such as promoters, polyadenylation signals, additional restriction enzyme sites, multiple cloning sites, other coding segments, and the like, such that their overall length may vary considerably. The nucleic acids can be any length. They can be, for example, 5, 10, 15, 20, 25, 30, 35, 40, 45, 50, 75, 100, 125, 175, 200, 250, 300, 350, 400, 450, 500, 750, 1000, 1500, 3000, 5000 or more nucleotides in length, and/or can comprise one or more additional sequences, for example, regulatory sequences, and/or be a part of a larger nucleic acid, for example, a vector. It is therefore contemplated that a nucleic acid fragment of almost any length may be employed, with the total length preferably being limited by the ease of preparation and use in the intended recombinant nucleic acid protocol. In some cases, a nucleic acid sequence may encode a polypeptide sequence with additional heterologous coding sequences, for example to allow for purification of the polypeptide, transport, secretion, post-translational modification, or for therapeutic benefits such as targeting or efficacy. As discussed above, a tag or other heterologous polypeptide may be added to the modified polypeptide-encoding sequence, wherein "heterologous" refers to a polypeptide that is not the same as the modified polypeptide.

The polypeptides, proteins, or polynucleotides encoding such polypeptides or proteins of the present invention as defined in the claims may include 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 (or any derivable range therein) or more variant amino acids or nucleic acid substitutions or be at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% (or any derivable range therein) similar, identical, or homologous with at least, or at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 300, 400, 500, 550, 1000 or more contiguous amino acids or nucleic acids, or any range derivable therein, of SEQ ID NOs:1-49.

In some embodiments of the present invention as defined in the claims, the protein or polypeptide may comprise amino acids 1 to 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303,304,305,306,307,308,309,310,311,312,313,314,315,316,317,318,319,320,321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, or 700 (or any derivable range therein) of SEQ ID NOs:1-14 or 34-40.

In some embodiments of the present invention as defined in the claims, the protein, polypeptide, or nucleic acid may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, or 700 (or any derivable range therein) contiguous amino acids of SEQ ID NOs: 1-49.

In some embodiments of the present invention as defined in the claims, the polypeptide, protein, or nucleic acid may comprise at least, at most, or exactly 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, or 700 (or any derivable range therein) contiguous amino acids of SEQ ID NOs:1-49 that are at least, at most, or exactly 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% (or any derivable range therein) similar, identical, or homologous with one of SEQ ID NOs:1-49.

In some embodiments of the present invention, there is a nucleic acid molecule or polypeptide starting at position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, or 700 of any of SEQ ID NOS:1-49 and comprising at least, at most, or exactly 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301,302,303,304,305,306,307,308,309,310,311,312,313,314,315,316,317,318,319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, or 700 (or any derivable range therein) contiguous amino acids or nucleotides of any of SEQ ID NOS:1-49.

The nucleotide as well as the protein, polypeptide, and peptide sequences for various genes have been previously disclosed, and may be found in the recognized computerized databases. Two commonly used databases are the National Center for Biotechnology Information's Genbank and GenPept databases (on the World Wide Web at ncbi.nlm.nih.gov/) and The Universal Protein Resource (UniProt; on the World Wide Web at uniprot.org). The coding regions for these genes may be amplified and/or expressed using the techniques disclosed herein or as would be known to those of ordinary skill in the art.

It is contemplated that in compositions, there is between about 0.001 mg and about 10 mg of total polypeptide, peptide, and/or protein per ml. The concentration of protein in a composition can be about, at least about or at most about 0.001, 0.010, 0.050, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0 mg/ml or more (or any range derivable therein).

In the case of proteins having catalytic activity (e.g., an enzyme), such a protein may be described using Enzyme Classification (EC) nomenclature. EC classifications of various enzymes have been previously disclosed, and may be found in recognized databases, for example, the ENZYME database (Bairoch A. The ENZYME database in 2000. Nucleic Acids Res. 2000 Jan 1;28(1):304-5. doi: 10.1093/nar/28.1.304).

### A. Signal Peptides

The present invention as defined in the claims is directed to synthetic signal peptides, and polynucleotides and nucleic acids encoding such signal peptides, and cells comprising such signal peptides, and methods for using cells in production and secretion of a protein (e.g., mammalian protein such as human milk protein). As used herein, "signal peptide" (or "signal peptide sequence") describes any peptide able to, when present at the N-terminal end of a newly synthesized polypeptide, direct the polypeptide across or into a cell membrane of a cell (e.g., the plasma membrane, the endoplasmic reticulum membrane, etc.). In some embodiments of the present invention as defined in the claims, a signal peptide is able to direct a polypeptide into a cell's secretory pathway and subsequent secretion of the polypeptide (described herein as a "secretion signal peptide").

As described herein, the present invention as defined in the claims relates to synthetic signal peptides comprising:
(a) a pre-region sequence from:
   (i) *P. pastoris* Ost1; or
   (ii) *P. pastoris* Pst1; and
(b) a pro-region sequence from:
   (i) *S. cerevisiae* mating factor α (MFα); or
   (ii) *P. pastoris* Epx1.

Certain signal peptides of the present invention as defined in the claims are described in Table 1 below.

**Table 1 - Signal peptides**

| **Description** | **Sequence** | **SEQ ID NO:** |
|---|---|---|
| SP1 (pre-Ost1 + pro-MFα) | MKFISILFLLIGSVFGAPVNTTTEDETAQIPAEAVIGYSDLEG DFDVAVLPFSNSTNNGLLFINTTIASIAAKEEGVSLEKREAE AYVEF | 1 |
| SP2 (pre-Pst1 + pro-MFα) | MQFGKVLFAISALAVTALGAPVNTTTEDETAQIPAEAVIGY SDLEGDFDVAVLPFSNSTNNGLLFINTTIASIAAKEEGVSLE KREAEAYVEF | 2 |
| SP3 (pre-Ost1 + pro-Epx1) | MKFISILFLLIGSVFGAPVAPAEEAANHLHKR | 3 |
| SP4 (pre-Pst1 + pro-Epx1) | MQFGKVLFAISALAVTALGAPVAPAEEAANHLHKR | 4 |
| SP1 (nucleic acid) | ATGAAATTCATCTCAATTCTGTTCCTTTTGATAGGCAGTG TATTTGGTGCTCCAGTCAACACTACAACAGAAGATGAAA CGGCACAAATTCCGGCTGAAGCTGTCATCGGTTACTCAG ATTTAGAAGGGGATTTCGATGTTGCTGTTTTGCCATTTTC CAACAGCACAAATAACGGGTTATTGTTTATAAATACTAC TATTGCCAGCATTGCTGCTAAAGAAGAAGGGGTATCTCT CGAGAAAAGAGAGGCTGAAGCTTATGTCGAGTTC | 41 |
| SP2 (nucleic acid) | ATGCAGTTTGGAAAGGTTCTATTTGCTATTTCTGCCCTGG CTGTCACAGCTCTGGGAGCTCCAGTCAACACTACAACAG AAGATGAAACGGCACAAATTCCGGCTGAAGCTGTCATC GGTTACTCAGATTTAGAAGGGGATTTCGATGTTGCTGTT TTGCCATTTTCCAACAGCACAAATAACGGGTTATTGTTT ATAAATACTACTATTGCCAGCATTGCTGCTAAAGAAGAA GGGGTATCTCTCGAGAAAAGAGAGGCTGAAGCTTATGTC GAGTTC | 42 |
| SP3 (nucleic acid) | ATGAAATTCATCTCAATTCTGTTCCTTTTGATAGGCAGTG TATTTGGTGCTCCAGTTGCTCCAGCCGAAGAGGCAGCAA ACCACTTGCACAAGCGT | 43 |
| SP4 (nucleic acid) | ATGCAGTTTGGAAAGGTTCTATTTGCTATTTCTGCCCTGG CTGTCACAGCTCTGGGAGCTCCAGTTGCTCCAGCCGAAG AGGCAGCAAACCACTTGCACAAGCGT | 44 |

The present invention as defined in the claims is related to nucleic acids encoding a polypeptides comprising a signal peptide as defined in the claims. Furthermore, the present invention as defined in the claims is related to cells expressing polypeptides comprising a signal peptide of the present invention as defined in the claims.

In some embodiments of the present invention as defined in the claims, a polypeptide comprises SEQ ID NO: 1. In some embodiments of the present invention as defined in the claims, a polypeptide comprises a sequence having at least 90%, 91%, 92%, 93%, 94%, 95%, 96%, 97%, 98%, or 99% identity to SEQ ID NO:1. In some embodiments of the present invention as defined in the claims, a polypeptide comprises a sequence having 1, 2, 3, 4, 5, 6, 7, 8, 9, or 10 amino acid substitutions (or more) relative to SEQ ID NO: 1.

In some embodiments of the present invention as defined in the claims, a polypeptide comprises SEQ ID NO:2. In some embodiments of the present invention as defined in the claims, a polypeptide comprises a sequence having at least 90%, 91%, 92%, 93%, 94%, 95%, 96%, 97%, 98%, or 99% identity to SEQ ID NO:2. In some embodiments of the present invention as defined in the claims, a polypeptide comprises a sequence having 1, 2, 3, 4, 5, 6, 7, 8, 9, or 10 amino acid substitutions (or more) relative to SEQ ID NO:2.

In some embodiments of the present invention as defined in the claims, a polypeptide comprises SEQ ID NO:3. In some embodiments of the present invention as defined in the claims, a polypeptide comprises a sequence having at least 90%, 91%, 92%, 93%, 94%, 95%, 96%, 97%, 98%, or 99% identity to SEQ ID NO:3. In some embodiments of the present invention as defined in the claims, a polypeptide comprises a sequence having 1, 2, 3, 4, 5, 6, 7, 8, 9, or 10 amino acid substitutions (or more) relative to SEQ ID NO:3.

In some embodiments of the present invention as defined in the claims, a polypeptide comprises SEQ ID NO:4. In some embodiments of the present invention as defined in the claims, a polypeptide comprises a sequence having at least 90%, 91%, 92%, 93%, 94%, 95%, 96%, 97%, 98%, or 99% identity to SEQ ID NO:4. In some embodiments of the present invention as defined in the claims, a polypeptide comprises a sequence having 1, 2, 3, 4, 5, 6, 7, 8, 9, or 10 amino acid substitutions (or more) relative to SEQ ID NO:4.

### B. Secretory Proteins

Embodiments of the present invention as defined in the claims include secretory proteins (also "secreted proteins"). As used herein, a "secretory protein" describes any protein secreted outside a cell. In certain cases, a secretory protein of the disclosure is a protein present in a human secretion, such as, for example, colostrum, milk, tears, seminal fluid, vaginal fluid, saliva, or other secretion. In some embodiments of the present invention as defined in the claims, a secretory protein is a human milk protein. In some embodiments of the present invention as defined in the claims, a secretory protein of the disclosure is not a human milk protein.

### 1. Human Milk Proteins

Embodiments of the present invention as defined in the claims include human milk proteins. Embodiments of the present invention as defined in the claims are related to cells expressing a human milk protein linked to a signal peptide of the present invention as defined in the claims (e.g., comprising SEQ ID NOs: 1, 2, 3, or 4). As used herein, a "human milk protein" describes any protein present in human breast milk. A human milk protein includes a protein derived from (e.g., isolated from) human breast milk, as well as any protein produced by other means (e.g., recombinant expression, chemical synthesis, etc.) having an amino acid sequence of a protein present in human breast milk. Various human milk proteins are recognized in the art and contemplated herein. Human milk proteins contemplated herein include, but are not limited to, secretory IgA (sIgA), human serum albumin, xanthine dehydrogenase, lactoferrin, lactoperoxidase, butyrophilin, lactadherin, adiponectin, β-casein, x-casein, leptin, lysozyme, and α-lactalbumin. In some embodiments of the present invention as defined in the claims, a human milk protein is a human whey protein. In some embodiments of the present invention as defined in the claims, a human milk protein is a recombinant human milk protein (e.g., produced by a non-mammalian cell such as a yeast cell).

Embodiments of the present invention as defined in the claims include human milk proteins having "human-like" glycans. Human-like glycans (also "human-like glycan structures") describe glycans having structures present in human glycoproteins. Such glycans include, for example, hybrid N-glycans, complex N-glycans, bi-antennary, tri-antennary, and tetra-antennary N-glycans, and glycans comprising sialic acid, galactose, N-acetylgalactosamine, or fucose. Human-like glycans include those having a Man3GlcNAc2 core structure. Accordingly, human milk proteins include those having one or more human-like glycans, for example hybrid N-glycans, complex N-glycans, bi-antennary N-glycans, tri-antennary N-glycans, tetra-antennary N-glycans, and combinations thereof.

Accordingly, in some embodiments of the present invention as defined in the claims include recombinant human milk proteins (e.g., recombinant human lactoferrin) comprising one or more human-like glycans. Such recombinant protein include, for example, those produced by engineered mammalian, fungal, yeast, bacterial, or other cells, including engineered cells described elsewhere herein. In certain embodiments of the present invention as defined in the claims, such recombinant proteins have a glycan pattern that different from a glycan pattern of a corresponding natural human milk protein. For example, in some embodiments of the present invention as defined in the claims, a recombinant human lactoferrin comprising one or more human-like glycans is included, where the lactoferrin has a glycan pattern that is different from a glycan pattern of any naturally occurring human lactoferrin (e.g., human lactoferrin in human breast milk).

### a. Lactoferrin

Embodiments of the present invention as defined in the claims include lactoferrin, as well as compositions comprising lactoferrin, including infant formula compositions. Embodiments of the present invention as defined in the claims are related to cells expressing human lactoferrin linked to a signal peptide (e.g., comprising SEQ ID NOs: 1, 2, 3, or 4). Lactoferrin (also "lactotransferrin") is a whey protein found in exocrine fluids such as breast milk and is encoded by the LTF gene. Without wishing to be bound by theory, lactoferrin is understood to have antimicrobial and anti-inflammatory properties. Embodiments of the present invention as defined in the claims include human lactoferrin (UniProtKB/Swiss-Prot accession number P02788), including isoforms thereof. The full sequence of human lactoferrin, including signal peptide, is provided as SEQ ID NO:34. The sequence of mature human lactoferrin following cleavage of the signal peptide is provided as SEQ ID NO:9.

**Table 2 - Human Lactoferrin sequences**

| **Protein** | **Sequence** | **SEQ ID NO** |
|---|---|---|
| Full length human lactoferrin | MKLVFLVLLFLGALGLCLAGRRRSVQWCAVSQPEATKCFQWQR NMRKVRGPPVSCIKRDSPIQCIQAIAENRADAVTLDGGFIYEAGL APYKLRPVAAEVYGTERQPRTHYYAVAVVKKGGSFQLNELQGL KSCHTGLRRTAGWNVPIGTLRPFLNWTGPPEPIEAAVARFFSASC VPGADKGQFPNLCRLCAGTGENKCAFSSQEPYFSYSGAFKCLRD GAGDVAFIRESTVFEDLSDEAERDEYELLCPDNTRKPVDKFKDC HLARVPSHAVVARSVNGKEDAIWNLLRQAQEKFGKDKSPKFQL FGSPSGQKDLLFKDSAIGFSRVPPRIDSGLYLGSGYFTAIQNLRKS EEEVAARRARVVWCAVGEQELRKCNQWSGLSEGSVTCSSASTT EDCIALVLKGEADAMSLDGGYVYTAGKCGLVPVLAENYKSQQS SDPDPNCVDRPVEGYLAVAVVRRSDTSLTWNSVKGKKSCHTAV DRTAGWNIPMGLLFNQTGSCKFDEYFSQSCAPGSDPRSNLCALCI GDEQGENKCVPNSNERYYGYTGAFRCLAENAGDVAFVKDVTVL | 34 |
| | QNTDGNNNEAWAKDLKLADFALLCLDGKRKPVTEARSCHLAM APNHAVVSRMDKVERLKQVLLHQQAKFGRNGSDCPDKFCLFQS ETKNLLFNDNTECLARLHGKTTYEKYLGPQYVAGITNLKKCSTS PLLEACEFLRK | |
| Mature human lactoferrin | GRRRSVQWCAVSQPEATKCFQWQRNMRKVRGPPVSCIKRDSPI QCIQAIAENRADAVTLDGGFIYEAGLAPYKLRPVAAEVYGTERQ PRTHYYAVAVVKKGGSFQLNELQGLKSCHTGLRRTAGWNVPIG TLRPFLNWTGPPEPIEAAVARFFSASCVPGADKGQFPNLCRLCAG TGENKCAFSSQEPYFSYSGAFKCLRDGAGDVAFIRESTVFEDLSD EAERDEYELLCPDNTRKPVDKFKDCHLARVPSHAVVARSVNGK EDAIWNLLRQAQEKFGKDKSPKFQLFGSPSGQKDLLFKDSAIGFS RVPPRIDSGLYLGSGYFTAIQNLRKSEEEVAARRARVVWCAVGE QELRKCNQWSGLSEGSVTCSSASTTEDCIALVLKGEADAMSLDG GYVYTAGKCGLVPVLAENYKSQQSSDPDPNCVDRPVEGYLAVA VVRRSDTSLTWNSVKGKKSCHTAVDRTAGWNIPMGLLFNQTGS CKFDEYFSQSCAPGSDPRSNLCALCIGDEQGENKCVPNSNERYY GYTGAFRCLAENAGDVAFVKDVTVLQNTDGNNNEAWAKDLKL ADFALLCLDGKRKPVTEARSCHLAMAPNHAVVSRMDKVERLK QVLLHQQAKFGRNGSDCPDKFCLFQSETKNLLFNDNTECLARLH GKTTYEKYLGPQYVAGITNLKKCSTSPLLEACEFLRK | 9 |
| SP 1-human lactoferrin | MKFISILFLLIGSVFGAPVNTTTEDETAQIPAEAVIGYSDLEGDFD VAVLPFSNSTNNGLLFINTTIASIAAKEEGVSLEKREAEAYVEFGR RRSVQWCAVSQPEATKCFQWQRNMRKVRGPPVSCIKRDSPIQCI QAIAENRADAVTLDGGFIYEAGLAPYKLRPVAAEVYGTERQPRT HYYAVAVVKKGGSFQLNELQGLKSCHTGLRRTAGWNVPIGTLR PFLNWTGPPEPIEAAVARFFSASCVPGADKGQFPNLCRLCAGTGE NKCAFSSQEPYFSYSGAFKCLRDGAGDVAFIRESTVFEDLSDEAE RDEYELLCPDNTRKPVDKFKDCHLARVPSHAVVARSVNGKEDAI WNLLRQAQEKFGKDKSPKFQLFGSPSGQKDLLFKDSAIGFSRVPP RIDSGLYLGSGYFTAIQNLRKSEEEVAARRARVVWCAVGEQELR KCNQWSGLSEGSVTCSSASTTEDCIALVLKGEADAMSLDGGYV YTAGKCGLVPVLAENYKSQQSSDPDPNCVDRPVEGYLAVAVVR RSDTSLTWNSVKGKKSCHTAVDRTAGWNIPMGLLFNQTGSCKF DEYFSQSCAPGSDPRSNLCALCIGDEQGENKCVPNSNERYYGYT GAFRCLAENAGDVAFVKDVTVLQNTDGNNNEAWAKDLKLADF ALLCLDGKRKPVTEARSCHLAMAPNHAVVSRMDKVERLKQVLL | 5 |
| | HQQAKFGRNGSDCPDKFCLFQSETKNLLFNDNTECLARLHGKTT YEKYLGPQYVAGITNLKKCSTSPLLEACEFLRK | |
| SP2-human lactoferrin | MQFGKVLFAISALAVTALGAPVNTTTEDETAQIPAEAVIGYSDLE GDFDVAVLPFSNSTNNGLLFINTTIASIAAKEEGVSLEKREAEAY VEFGRRRSVQWCAVSQPEATKCFQWQRNMRKVRGPPVSCIKRD SPIQCIQAIAENRADAVTLDGGFIYEAGLAPYKLRPVAAEVYGTE RQPRTHYYAVAVVKKGGSFQLNELQGLKSCHTGLRRTAGWNVP IGTLRPFLNWTGPPEPIEAAVARFFSASCVPGADKGQFPNLCRLC AGTGENKCAFSSQEPYFSYSGAFKCLRDGAGDVAFIRESTVFEDL SDEAERDEYELLCPDNTRKPVDKFKDCHLARVPSHAVVARSVN GKEDAIWNLLRQAQEKFGKDKSPKFQLFGSPSGQKDLLFKDSAI GFSRVPPRIDSGLYLGSGYFTAIQNLRKSEEEVAARRARVVWCA VGEQELRKCNQWSGLSEGSVTCSSASTTEDCIALVLKGEADAMS LDGGYVYTAGKCGLVPVLAENYKSQQSSDPDPNCVDRPVEGYL AVAVVRRSDTSLTWNSVKGKKSCHTAVDRTAGWNIPMGLLFNQ TGSCKFDEYFSQSCAPGSDPRSNLCALCIGDEQGENKCVPNSNER YYGYTGAFRCLAENAGDVAFVKDVTVLQNTDGNNNEAWAKDL KLADFALLCLDGKRKPVTEARSCHLAMAPNHAVVSRMDKVERL KQVLLHQQAKFGRNGSDCPDKFCLFQSETKNLLFNDNTECLARL HGKTTYEKYLGPQYVAGITNLKKCSTSPLLEACEFLRK | 6 |
| SP3-human lactoferrin | MKFISILFLLIGSVFGAPVAPAEEAANHLHKRGRRRSVQWCAVSQ PEATKCFQWQRNMRKVRGPPVSCIKRDSPIQCIQAIAENRADAVT LDGGFIYEAGLAPYKLRPVAAEVYGTERQPRTHYYAVAVVKKG GSFQLNELQGLKSCHTGLRRTAGWNVPIGTLRPFLNWTGPPEPIE AAVARFFSASCVPGADKGQFPNLCRLCAGTGENKCAFSSQEPYF SYSGAFKCLRDGAGDVAFIRESTVFEDLSDEAERDEYELLCPDNT RKPVDKFKDCHLARVPSHAVVARSVNGKEDAIWNLLRQAQEKF GKDKSPKFQLFGSPSGQKDLLFKDSAIGFSRVPPRIDSGLYLGSGY FTAIQNLRKSEEEVAARRARVVWCAVGEQELRKCNQWSGLSEG SVTCSSASTTEDCIALVLKGEADAMSLDGGYVYTAGKCGLVPVL AENYKSQQSSDPDPNCVDRPVEGYLAVAVVRRSDTSLTWNSVK GKKSCHTAVDRTAGWNIPMGLLFNQTGSCKFDEYFSQSCAPGSD PRSNLCALCIGDEQGENKCVPNSNERYYGYTGAFRCLAENAGDV AFVKDVTVLQNTDGNNNEAWAKDLKLADFALLCLDGKRKPVT EARSCHLAMAPNHAVVSRMDKVERLKQVLLHQQAKFGRNGSD | 7 |
| | CPDKFCLFQSETKNLLFNDNTECLARLHGKTTYEKYLGPQYVAG ITNLKKCSTSPLLEACEFLRK | |
| SP4-human lactoferrin | MQFGKVLFAISALAVTALGAPVAPAEEAANHLHKRGRRRSVQW CAVSQPEATKCFQWQRNMRKVRGPPVSCIKRDSPIQCIQAIAENR ADAVTLDGGFIYEAGLAPYKLRPVAAEVYGTERQPRTHYYAVA VVKKGGSFQLNELQGLKSCHTGLRRTAGWNVPIGTLRPFLNWT GPPEPIEAAVARFFSASCVPGADKGQFPNLCRLCAGTGENKCAFS SQEPYFSYSGAFKCLRDGAGDVAFIRESTVFEDLSDEAERDEYEL LCPDNTRKPVDKFKDCHLARVPSHAVVARSVNGKEDAIWNLLR QAQEKFGKDKSPKFQLFGSPSGQKDLLFKDSAIGFSRVPPRIDSGL YLGSGYFTAIQNLRKSEEEVAARRARVVWCAVGEQELRKCNQW SGLSEGSVTCSSASTTEDCIALVLKGEADAMSLDGGYVYTAGKC GLVPVLAENYKSQQSSDPDPNCVDRPVEGYLAVAVVRRSDTSLT WNSVKGKKSCHTAVDRTAGWNIPMGLLFNQTGSCKFDEYFSQS CAPGSDPRSNLCALCIGDEQGENKCVPNSNERYYGYTGAFRCLA ENAGDVAFVKDVTVLQNTDGNNNEAWAKDLKLADFALLCLDG KRKPVTEARSCHLAMAPNHAVVSRMDKVERLKQVLLHQQAKF GRNGSDCPDKFCLFQSETKNLLFNDNTECLARLHGKTTYEKYLG PQYVAGITNLKKCSTSPLLEACEFLRK | 8 |
| *P. pastoris* codon-optimized Human lactoferrin gene | GCCGGAAGAAGAAGAAGTGTTCAATGGTGCGCCGTTAGTCAA CCTGAGGCTACAAAGTGTTTTCAATGGCAGAGAAATATGAGA AAGGTTAGAGGTCCACCTGTTTCTTGTATCAAGAGAGATTCTC CAATCCAATGTATTCAAGCTATTGCTGAGAACAGAGCTGATG CTGTTACTTTGGATGGTGGTTTTATCTACGAAGCTGGTTTGGC TCCATATAAACTTAGACCAGTTGCTGCTGAGGTTTACGGTACT GAAAGACAACCTAGAACTCATTACTATGCTGTTGCTGTTGTTA AGAAAGGTGGTTCTTTCCAATTGAACGAATTGCAAGGTTTGA AGTCTTGTCACACTGGTTTGAGAAGAACTGCTGGTTGGAATGT TCCAATTGGTACTTTAAGACCATTTCTTAACTGGACTGGTCCA CCTGAGCCAATTGAAGCTGCTGTTGCTAGATTTTTCTCTGCTTC TTGTGTTCCAGGTGCTGATAAGGGTCAATTTCCTAATTTGTGT AGATTGTGTGCTGGTACTGGAGAGAACAAATGTGCTTTCTCTT CTCAAGAACCTTACTTTTCTTATTCTGGTGCTTTCAAGTGTTTG AGAGATGGTGCTGGAGATGTTGCTTTTATTAGAGAGTCTACTG TTTTCGAAGATTTGTCTGATGAGGCTGAAAGAGATGAGTATG AATTGTTGTGTCCAGATAACACTAGAAAGCCTGTTGATAAGTT | 45 |
| | TAAAGATTGTCATTTGGCTAGAGTTCCATCTCACGCTGTTGTT GCTAGATCTGTTAATGGTAAAGAGGATGCTATTTGGAACTTGT TGAGACAAGCTCAAGAAAAGTTCGGTAAAGACAAGTCTCCAA AGTTCCAATTGTTCGGTTCTCCTTCTGGTCAAAAGGATTTGTT GTTTAAAGATTCTGCTATCGGTTTCTCTAGAGTTCCACCTAGA ATTGATTCTGGTTTGTACTTGGGTTCTGGTTACTTCACTGCTAT CCAAAATTTGAGAAAGTCTGAAGAGGAAGTTGCTGCTAGAAG AGCTAGAGTTGTTTGGTGTGCTGTTGGAGAGCAAGAATTGAG AAAGTGTAACCAATGGTCTGGTTTGTCTGAAGGTTCTGTTACT TGTTCTTCTGCTTCTACTACTGAGGATTGTATTGCTTTGGTTTT GAAAGGTGAAGCTGATGCTATGTCTTTGGATGGTGGTTACGTT TATACTGCTGGTAAATGTGGTTTGGTTCCAGTTTTGGCTGAGA ATTACAAATCTCAACAATCTTCTGATCCAGATCCTAACTGTGT TGATAGACCTGTTGAAGGTTATTTGGCTGTTGCTGTTGTTAGA AGATCTGATACTTCTTTGACTTGGAACTCTGTTAAAGGTAAAA AGTCTTGTCATACTGCTGTTGATAGAACCGCCGGTTGGAATAT TCCAATGGGTTTGTTGTTTAACCAAACTGGTTCTTGTAAGTTT GATGAGTACTTCTCTCAATCTTGTGCTCCAGGTTCTGATCCTA GATCTAATTTGTGTGCTTTGTGTATTGGAGATGAGCAAGGTGA AAACAAATGTGTTCCTAATTCTAACGAGAGATACTATGGTTAT ACTGGTGCTTTTAGATGTTTGGCTGAAAACGCCGGAGATGTTG CTTTCGTTAAGGATGTTACTGTTTTGCAAAACACTGATGGTAA CAATAACGAAGCTTGGGCTAAGGATTTGAAATTGGCTGATTTC GCTTTGTTGTGTTTGGATGGTAAAAGAAAACCAGTTACTGAGG CTAGATCTTGTCATTTGGCTATGGCTCCTAACCACGCTGTTGTT TCTAGAATGGATAAGGTTGAAAGATTGAAGCAAGTTTTGTTG CATCAACAGGCTAAGTTTGGTAGAAATGGTTCTGATTGTCCTG ATAAGTTTTGTTTGTTCCAATCTGAGACTAAAAACTTGTTGTT CAATGATAACACTGAATGTTTGGCTAGATTGCACGGTAAAAC TACTTACGAAAAATATTTGGGTCCTCAATACGTTGCTGGTATT ACTAACTTGAAGAAATGCTCCACCAGTCCATTGCTTGAGGCTT GCGAGTTCCTTAGAAAATAA | |
| SP1-codon optimized hLF gene | ATGAAATTCATCTCAATTCTGTTCCTTTTGATAGGCAGTGTATT TGGTGCTCCAGTCAACACTACAACAGAAGATGAAACGGCACA AATTCCGGCTGAAGCTGTCATCGGTTACTCAGATTTAGAAGGG GATTTCGATGTTGCTGTTTTGCCATTTTCCAACAGCACAAATA | 46 |
| | ACGGGTTATTGTTTATAAATACTACTATTGCCAGCATTGCTGC TAAAGAAGAAGGGGTATCTCTCGAGAAAAGAGAGGCTGAAG CTTATGTCGAGTTCGCCGGAAGAAGAAGTGTTCAATGGT GCGCCGTTAGTCAACCTGAGGCTACAAAGTGTTTTCAATGGCA GAGAAATATGAGAAAGGTTAGAGGTCCACCTGTTTCTTGTATC AAGAGAGATTCTCCAATCCAATGTATTCAAGCTATTGCTGAGA ACAGAGCTGATGCTGTTACTTTGGATGGTGGTTTTATCTACGA AGCTGGTTTGGCTCCATATAAACTTAGACCAGTTGCTGCTGAG GTTTACGGTACTGAAAGACAACCTAGAACTCATTACTATGCTG TTGCTGTTGTTAAGAAAGGTGGTTCTTTCCAATTGAACGAATT GCAAGGTTTGAAGTCTTGTCACACTGGTTTGAGAAGAACTGCT GGTTGGAATGTTCCAATTGGTACTTTAAGACCATTTCTTAACT GGACTGGTCCACCTGAGCCAATTGAAGCTGCTGTTGCTAGATT TTTCTCTGCTTCTTGTGTTCCAGGTGCTGATAAGGGTCAATTTC CTAATTTGTGTAGATTGTGTGCTGGTACTGGAGAGAACAAATG TGCTTTCTCTTCTCAAGAACCTTACTTTTCTTATTCTGGTGCTT TCAAGTGTTTGAGAGATGGTGCTGGAGATGTTGCTTTTATTAG AGAGTCTACTGTTTTCGAAGATTTGTCTGATGAGGCTGAAAGA GATGAGTATGAATTGTTGTGTCCAGATAACACTAGAAAGCCT GTTGATAAGTTTAAAGATTGTCATTTGGCTAGAGTTCCATCTC ACGCTGTTGTTGCTAGATCTGTTAATGGTAAAGAGGATGCTAT TTGGAACTTGTTGAGACAAGCTCAAGAAAAGTTCGGTAAAGA CAAGTCTCCAAAGTTCCAATTGTTCGGTTCTCCTTCTGGTCAA AAGGATTTGTTGTTTAAAGATTCTGCTATCGGTTTCTCTAGAG TTCCACCTAGAATTGATTCTGGTTTGTACTTGGGTTCTGGTTAC TTCACTGCTATCCAAAATTTGAGAAAGTCTGAAGAGGAAGTT GCTGCTAGAAGAGCTAGAGTTGTTTGGTGTGCTGTTGGAGAG CAAGAATTGAGAAAGTGTAACCAATGGTCTGGTTTGTCTGAA GGTTCTGTTACTTGTTCTTCTGCTTCTACTACTGAGGATTGTAT TGCTTTGGTTTTGAAAGGTGAAGCTGATGCTATGTCTTTGGAT GGTGGTTACGTTTATACTGCTGGTAAATGTGGTTTGGTTCCAG TTTTGGCTGAGAATTACAAATCTCAACAATCTTCTGATCCAGA TCCTAACTGTGTTGATAGACCTGTTGAAGGTTATTTGGCTGTT GCTGTTGTTAGAAGATCTGATACTTCTTTGACTTGGAACTCTG TTAAAGGTAAAAAGTCTTGTCATACTGCTGTTGATAGAACCGC CGGTTGGAATATTCCAATGGGTTTGTTGTTTAACCAAACTGGT | |
| | TCTTGTAAGTTTGATGAGTACTTCTCTCAATCTTGTGCTCCAGG TTCTGATCCTAGATCTAATTTGTGTGCTTTGTGTATTGGAGATG AGCAAGGTGAAAACAAATGTGTTCCTAATTCTAACGAGAGAT ACTATGGTTATACTGGTGCTTTTAGATGTTTGGCTGAAAACGC CGGAGATGTTGCTTTCGTTAAGGATGTTACTGTTTTGCAAAAC ACTGATGGTAACAATAACGAAGCTTGGGCTAAGGATTTGAAA TTGGCTGATTTCGCTTTGTTGTGTTTGGATGGTAAAAGAAAAC CAGTTACTGAGGCTAGATCTTGTCATTTGGCTATGGCTCCTAA CCACGCTGTTGTTTCTAGAATGGATAAGGTTGAAAGATTGAA GCAAGTTTTGTTGCATCAACAGGCTAAGTTTGGTAGAAATGGT TCTGATTGTCCTGATAAGTTTTGTTTGTTCCAATCTGAGACTAA AAACTTGTTGTTCAATGATAACACTGAATGTTTGGCTAGATTG CACGGTAAAACTACTTACGAAAAATATTTGGGTCCTCAATAC GTTGCTGGTATTACTAACTTGAAGAAATGCTCCACCAGTCCAT TGCTTGAGGCTTGCGAGTTCCTTAGAAAATAA | |
| SP2-codon optimized hLF gene | ATGCAGTTTGGAAAGGTTCTATTTGCTATTTCTGCCCTGGCTG TCACAGCTCTGGGAGCTCCAGTCAACACTACAACAGAAGATG AAACGGCACAAATTCCGGCTGAAGCTGTCATCGGTTACTCAG ATTTAGAAGGGGATTTCGATGTTGCTGTTTTGCCATTTTCCAA CAGCACAAATAACGGGTTATTGTTTATAAATACTACTATTGCC AGCATTGCTGCTAAAGAAGAAGGGGTATCTCTCGAGAAAAGA GAGGCTGAAGCTTATGTCGAGTTCGCCGGAAGAAGAAGAAGT GTTCAATGGTGCGCCGTTAGTCAACCTGAGGCTACAAAGTGTT TTCAATGGCAGAGAAATATGAGAAAGGTTAGAGGTCCACCTG TTTCTTGTATCAAGAGAGATTCTCCAATCCAATGTATTCAAGC TATTGCTGAGAACAGAGCTGATGCTGTTACTTTGGATGGTGGT TTTATCTACGAAGCTGGTTTGGCTCCATATAAACTTAGACCAG TTGCTGCTGAGGTTTACGGTACTGAAAGACAACCTAGAACTC ATTACTATGCTGTTGCTGTTGTTAAGAAAGGTGGTTCTTTCCA ATTGAACGAATTGCAAGGTTTGAAGTCTTGTCACACTGGTTTG AGAAGAACTGCTGGTTGGAATGTTCCAATTGGTACTTTAAGAC CATTTCTTAACTGGACTGGTCCACCTGAGCCAATTGAAGCTGC TGTTGCTAGATTTTTCTCTGCTTCTTGTGTTCCAGGTGCTGATA AGGGTCAATTTCCTAATTTGTGTAGATTGTGTGCTGGTACTGG AGAGAACAAATGTGCTTTCTCTTCTCAAGAACCTTACTTTTCT TATTCTGGTGCTTTCAAGTGTTTGAGAGATGGTGCTGGAGATG | 47 |
| | TTGCTTTTATTAGAGAGTCTACTGTTTTCGAAGATTTGTCTGAT GAGGCTGAAAGAGATGAGTATGAATTGTTGTGTCCAGATAAC ACTAGAAAGCCTGTTGATAAGTTTAAAGATTGTCATTTGGCTA GAGTTCCATCTCACGCTGTTGTTGCTAGATCTGTTAATGGTAA AGAGGATGCTATTTGGAACTTGTTGAGACAAGCTCAAGAAAA GTTCGGTAAAGACAAGTCTCCAAAGTTCCAATTGTTCGGTTCT CCTTCTGGTCAAAAGGATTTGTTGTTTAAAGATTCTGCTATCG GTTTCTCTAGAGTTCCACCTAGAATTGATTCTGGTTTGTACTTG GGTTCTGGTTACTTCACTGCTATCCAAAATTTGAGAAAGTCTG AAGAGGAAGTTGCTGCTAGAAGAGCTAGAGTTGTTTGGTGTG CTGTTGGAGAGCAAGAATTGAGAAAGTGTAACCAATGGTCTG GTTTGTCTGAAGGTTCTGTTACTTGTTCTTCTGCTTCTACTACT GAGGATTGTATTGCTTTGGTTTTGAAAGGTGAAGCTGATGCTA TGTCTTTGGATGGTGGTTACGTTTATACTGCTGGTAAATGTGG TTTGGTTCCAGTTTTGGCTGAGAATTACAAATCTCAACAATCT TCTGATCCAGATCCTAACTGTGTTGATAGACCTGTTGAAGGTT ATTTGGCTGTTGCTGTTGTTAGAAGATCTGATACTTCTTTGACT TGGAACTCTGTTAAAGGTAAAAAGTCTTGTCATACTGCTGTTG ATAGAACCGCCGGTTGGAATATTCCAATGGGTTTGTTGTTTAA CCAAACTGGTTCTTGTAAGTTTGATGAGTACTTCTCTCAATCTT GTGCTCCAGGTTCTGATCCTAGATCTAATTTGTGTGCTTTGTGT ATTGGAGATGAGCAAGGTGAAAACAAATGTGTTCCTAATTCT AACGAGAGATACTATGGTTATACTGGTGCTTTTAGATGTTTGG CTGAAAACGCCGGAGATGTTGCTTTCGTTAAGGATGTTACTGT TTTGCAAAACACTGATGGTAACAATAACGAAGCTTGGGCTAA GGATTTGAAATTGGCTGATTTCGCTTTGTTGTGTTTGGATGGT AAAAGAAAACCAGTTACTGAGGCTAGATCTTGTCATTTGGCT ATGGCTCCTAACCACGCTGTTGTTTCTAGAATGGATAAGGTTG AAAGATTGAAGCAAGTTTTGTTGCATCAACAGGCTAAGTTTG GTAGAAATGGTTCTGATTGTCCTGATAAGTTTTGTTTGTTCCA ATCTGAGACTAAAAACTTGTTGTTCAATGATAACACTGAATGT TTGGCTAGATTGCACGGTAAAACTACTTACGAAAAATATTTGG GTCCTCAATACGTTGCTGGTATTACTAACTTGAAGAAATGCTC CACCAGTCCATTGCTTGAGGCTTGCGAGTTCCTTAGAAAATAA | |
| SP3-codon optimized hLF gene | ATGAAATTCATCTCAATTCTGTTCCTTTTGATAGGCAGTGTATT TGGTGCTCCAGTTGCTCCAGCCGAAGAGGCAGCAAACCACTT GCACAAGCGTGCCGGAAGAAGAAGTGTTCAATGGTGCGC CGTTAGTCAACCTGAGGCTACAAAGTGTTTTCAATGGCAGAG AAATATGAGAAAGGTTAGAGGTCCACCTGTTTCTTGTATCAAG AGAGATTCTCCAATCCAATGTATTCAAGCTATTGCTGAGAACA GAGCTGATGCTGTTACTTTGGATGGTGGTTTTATCTACGAAGC TGGTTTGGCTCCATATAAACTTAGACCAGTTGCTGCTGAGGTT TACGGTACTGAAAGACAACCTAGAACTCATTACTATGCTGTTG CTGTTGTTAAGAAAGGTGGTTCTTTCCAATTGAACGAATTGCA AGGTTTGAAGTCTTGTCACACTGGTTTGAGAAGAACTGCTGGT TGGAATGTTCCAATTGGTACTTTAAGACCATTTCTTAACTGGA CTGGTCCACCTGAGCCAATTGAAGCTGCTGTTGCTAGATTTTT CTCTGCTTCTTGTGTTCCAGGTGCTGATAAGGGTCAATTTCCT AATTTGTGTAGATTGTGTGCTGGTACTGGAGAGAACAAATGT GCTTTCTCTTCTCAAGAACCTTACTTTTCTTATTCTGGTGCTTT CAAGTGTTTGAGAGATGGTGCTGGAGATGTTGCTTTTATTAGA GAGTCTACTGTTTTCGAAGATTTGTCTGATGAGGCTGAAAGAG ATGAGTATGAATTGTTGTGTCCAGATAACACTAGAAAGCCTGT TGATAAGTTTAAAGATTGTCATTTGGCTAGAGTTCCATCTCAC GCTGTTGTTGCTAGATCTGTTAATGGTAAAGAGGATGCTATTT GGAACTTGTTGAGACAAGCTCAAGAAAAGTTCGGTAAAGACA AGTCTCCAAAGTTCCAATTGTTCGGTTCTCCTTCTGGTCAAAA GGATTTGTTGTTTAAAGATTCTGCTATCGGTTTCTCTAGAGTTC CACCTAGAATTGATTCTGGTTTGTACTTGGGTTCTGGTTACTTC ACTGCTATCCAAAATTTGAGAAAGTCTGAAGAGGAAGTTGCT GCTAGAAGAGCTAGAGTTGTTTGGTGTGCTGTTGGAGAGCAA GAATTGAGAAAGTGTAACCAATGGTCTGGTTTGTCTGAAGGTT CTGTTACTTGTTCTTCTGCTTCTACTACTGAGGATTGTATTGCT TTGGTTTTGAAAGGTGAAGCTGATGCTATGTCTTTGGATGGTG GTTACGTTTATACTGCTGGTAAATGTGGTTTGGTTCCAGTTTTG GCTGAGAATTACAAATCTCAACAATCTTCTGATCCAGATCCTA ACTGTGTTGATAGACCTGTTGAAGGTTATTTGGCTGTTGCTGT TGTTAGAAGATCTGATACTTCTTTGACTTGGAACTCTGTTAAA GGTAAAAAGTCTTGTCATACTGCTGTTGATAGAACCGCCGGTT GGAATATTCCAATGGGTTTGTTGTTTAACCAAACTGGTTCTTG | 48 |
| | TAAGTTTGATGAGTACTTCTCTCAATCTTGTGCTCCAGGTTCTG ATCCTAGATCTAATTTGTGTGCTTTGTGTATTGGAGATGAGCA AGGTGAAAACAAATGTGTTCCTAATTCTAACGAGAGATACTA TGGTTATACTGGTGCTTTTAGATGTTTGGCTGAAAACGCCGGA GATGTTGCTTTCGTTAAGGATGTTACTGTTTTGCAAAACACTG ATGGTAACAATAACGAAGCTTGGGCTAAGGATTTGAAATTGG CTGATTTCGCTTTGTTGTGTTTGGATGGTAAAAGAAAACCAGT TACTGAGGCTAGATCTTGTCATTTGGCTATGGCTCCTAACCAC GCTGTTGTTTCTAGAATGGATAAGGTTGAAAGATTGAAGCAA GTTTTGTTGCATCAACAGGCTAAGTTTGGTAGAAATGGTTCTG ATTGTCCTGATAAGTTTTGTTTGTTCCAATCTGAGACTAAAAA CTTGTTGTTCAATGATAACACTGAATGTTTGGCTAGATTGCAC GGTAAAACTACTTACGAAAAATATTTGGGTCCTCAATACGTTG CTGGTATTACTAACTTGAAGAAATGCTCCACCAGTCCATTGCT TGAGGCTTGCGAGTTCCTTAGAAAATAA | |
| SP4-codon optimized hLF gene | ATGCAGTTTGGAAAGGTTCTATTTGCTATTTCTGCCCTGGCTG TCACAGCTCTGGGAGCTCCAGTTGCTCCAGCCGAAGAGGCAG CAAACCACTTGCACAAGCGTGCCGGAAGAAGAAGTGTTC AATGGTGCGCCGTTAGTCAACCTGAGGCTACAAAGTGTTTTCA ATGGCAGAGAAATATGAGAAAGGTTAGAGGTCCACCTGTTTC TTGTATCAAGAGAGATTCTCCAATCCAATGTATTCAAGCTATT GCTGAGAACAGAGCTGATGCTGTTACTTTGGATGGTGGTTTTA TCTACGAAGCTGGTTTGGCTCCATATAAACTTAGACCAGTTGC TGCTGAGGTTTACGGTACTGAAAGACAACCTAGAACTCATTA CTATGCTGTTGCTGTTGTTAAGAAAGGTGGTTCTTTCCAATTG AACGAATTGCAAGGTTTGAAGTCTTGTCACACTGGTTTGAGAA GAACTGCTGGTTGGAATGTTCCAATTGGTACTTTAAGACCATT TCTTAACTGGACTGGTCCACCTGAGCCAATTGAAGCTGCTGTT GCTAGATTTTTCTCTGCTTCTTGTGTTCCAGGTGCTGATAAGG GTCAATTTCCTAATTTGTGTAGATTGTGTGCTGGTACTGGAGA GAACAAATGTGCTTTCTCTTCTCAAGAACCTTACTTTTCTTATT CTGGTGCTTTCAAGTGTTTGAGAGATGGTGCTGGAGATGTTGC TTTTATTAGAGAGTCTACTGTTTTCGAAGATTTGTCTGATGAG GCTGAAAGAGATGAGTATGAATTGTTGTGTCCAGATAACACT AGAAAGCCTGTTGATAAGTTTAAAGATTGTCATTTGGCTAGAG TTCCATCTCACGCTGTTGTTGCTAGATCTGTTAATGGTAAAGA | 49 |
| | GGATGCTATTTGGAACTTGTTGAGACAAGCTCAAGAAAAGTT CGGTAAAGACAAGTCTCCAAAGTTCCAATTGTTCGGTTCTCCT TCTGGTCAAAAGGATTTGTTGTTTAAAGATTCTGCTATCGGTT TCTCTAGAGTTCCACCTAGAATTGATTCTGGTTTGTACTTGGG TTCTGGTTACTTCACTGCTATCCAAAATTTGAGAAAGTCTGAA GAGGAAGTTGCTGCTAGAAGAGCTAGAGTTGTTTGGTGTGCT GTTGGAGAGCAAGAATTGAGAAAGTGTAACCAATGGTCTGGT TTGTCTGAAGGTTCTGTTACTTGTTCTTCTGCTTCTACTACTGA GGATTGTATTGCTTTGGTTTTGAAAGGTGAAGCTGATGCTATG TCTTTGGATGGTGGTTACGTTTATACTGCTGGTAAATGTGGTT TGGTTCCAGTTTTGGCTGAGAATTACAAATCTCAACAATCTTC TGATCCAGATCCTAACTGTGTTGATAGACCTGTTGAAGGTTAT TTGGCTGTTGCTGTTGTTAGAAGATCTGATACTTCTTTGACTTG GAACTCTGTTAAAGGTAAAAAGTCTTGTCATACTGCTGTTGAT AGAACCGCCGGTTGGAATATTCCAATGGGTTTGTTGTTTAACC AAACTGGTTCTTGTAAGTTTGATGAGTACTTCTCTCAATCTTGT GCTCCAGGTTCTGATCCTAGATCTAATTTGTGTGCTTTGTGTAT TGGAGATGAGCAAGGTGAAAACAAATGTGTTCCTAATTCTAA CGAGAGATACTATGGTTATACTGGTGCTTTTAGATGTTTGGCT GAAAACGCCGGAGATGTTGCTTTCGTTAAGGATGTTACTGTTT TGCAAAACACTGATGGTAACAATAACGAAGCTTGGGCTAAGG ATTTGAAATTGGCTGATTTCGCTTTGTTGTGTTTGGATGGTAA AAGAAAACCAGTTACTGAGGCTAGATCTTGTCATTTGGCTATG GCTCCTAACCACGCTGTTGTTTCTAGAATGGATAAGGTTGAAA GATTGAAGCAAGTTTTGTTGCATCAACAGGCTAAGTTTGGTAG AAATGGTTCTGATTGTCCTGATAAGTTTTGTTTGTTCCAATCTG AGACTAAAAACTTGTTGTTCAATGATAACACTGAATGTTTGGC TAGATTGCACGGTAAAACTACTTACGAAAAATATTTGGGTCCT CAATACGTTGCTGGTATTACTAACTTGAAGAAATGCTCCACCA GTCCATTGCTTGAGGCTTGCGAGTTCCTTAGAAAATAA | |

In some embodiments of the present invention as defined in the claims, a human lactoferrin is a recombinant human lactoferrin (rhLactoferrin). In some embodiments of the present invention as defined in the claims, a recombinant human lactoferrin is obtained from a mammalian, fungal, yeast, bacterial, or other cell. In some embodiments of the present invention as defined in the claims, a recombinant human lactoferrin is not obtained from a mammalian cell. In certain embodiments of the present invention as defined in the claims, a recombinant human lactoferrin is obtained from a fungal cell. The fungal cell may be, for example, a *Arxula, Aspegillus, Aurantiochytrium, Candida, Claviceps, Cryptococcus, Cunninghamella, Geotrichum, Hansenula, Kluyveromyces, Kodamaea, Komagataella, Leucosporidiella, Lipomyces, Mortierella, Ogataea, Pichia, Prototheca, Rhizopus, Rhodosporidium, Rhodotorula, Saccharomyces, Schizosaccharomyces, Tremella, Trichosporon, Wickerhamomyces,* or *Yarrowia* cell. In some embodiments of the present invention as defined in the claims, the fungal cell is a yeast cell. In some embodiments of the present invention as defined in the claims, the yeast cell is yeast cell is a *Komagataella* cell (e.g., *Komagataella phaffii, Komagataella pastoris, Komagataella pseudopastoris*)*.* Additional cells suitable for recombinant protein production are recognized in the art and contemplated herein. In some embodiments of the present invention as defined in the claims, a recombinant human lactoferrin is obtained from a bacterial cell. In other embodiments of the present invention as defined in the claims, a human lactoferrin is isolated from a natural source.

Particular embodiments of the present invention as defined in the claims include human lactoferrin having at least one hybrid or complex N-glycan. In some embodiments of the present invention as defined in the claims, the human lactoferrin comprises a glycan comprising one or more of sialic acid, galactose, N-acetylgalactosamine, or fucose. In some embodiments of the present invention as defined in the claims, the human lactoferrin comprises a bi-antennary, tri-antennary, or tetra-antennary N-glycan. Human lactoferrin having one or more hybrid, complex, bi-antennary, tri-antennary, or tetra-antennary N-glycan may be useful in, for example, infant formula or other nutritional compositions or supplements.

### b. Alpha-lactalbumin (α-lactalbumin)

Embodiments of the present invention as defined in the claims include alpha-lactalbumin, as well as compositions comprising alpha-lactalbumin, including infant formula compositions. Embodiments of the present invention as defined in the claims, are related to cells expressing human alpha-lactalbumin linked to a signal peptide (e.g., comprising SEQ ID NOs: 1, 2, 3, or 4). Alpha-lactalbumin (also "α-lactalbumin") is a whey protein found in breast milk and is encoded by the LALBA gene. Certain embodiments of the present invention as defined in the claimsinclude human α-lactalbumin (UniProtKB/Swiss-Prot accession number P00709), including isoforms thereof. The full sequence of human α-lactalbumin, including signal peptide, is provided as SEQ ID NO:36. The sequence of mature human α-lactalbumin following cleavage of the signal peptide is provided as SEQ ID NO:35.

**Table 3 - Human Lactoferrin sequences**

| **Protein** | **Sequence** | **SEQ ID NO** |
|---|---|---|
| Full length human α-lactalbumin | MRFFVPLFLVGILFPAILAKQFTKCELSQLLKDIDGYGGIALPELICT MFHTSGYDTQAIVENNESTEYGLFQISNKLWCKSSQVPQSRNICDIS CDKFLDDDITDDIMCAKKILDIKGIDYWLAHKALCTEKLEQWLCE KL | 36 |
| Mature human α-lactalbumin | KQFTKCELSQLLKDIDGYGGIALPELICTMFHTSGYDTQAIVENNES TEYGLFQISNKLWCKSSQVPQSRNICDISCDKFLDDDITDDIMCAK KILDIKGIDYWLAHKALCTEKLEQWLCEKL | 35 |
| SP 1-human α-lactalbumin | MKFISILFLLIGSVFGAPVNTTTEDETAQIPAEAVIGYSDLEGDFDVA VLPFSNSTNNGLLFINTTIASIAAKEEGVSLEKREAEAYVEFKQFTK CELSQLLKDIDGYGGIALPELICTMFHTSGYDTQAIVENNESTEYGL FQISNKLWCKSSQVPQSRNICDISCDKFLDDDITDDIMCAKKILDIK GIDYWLAHKALCTEKLEQWLCEKL | 37 |
| SP2-human α-lactalbumin | MQFGKVLFAISALAVTALGAPVNTTTEDETAQIPAEAVIGYSDLEG DFDVAVLPFSNSTNNGLLFINTTIASIAAKEEGVSLEKREAEAYVEF KQFTKCELSQLLKDIDGYGGIALPELICTMFHTSGYDTQAIVENNES TEYGLFQISNKLWCKSSQVPQSRNICDISCDKFLDDDITDDIMCAK KILDIKGIDYWLAHKALCTEKLEQWLCEKL | 38 |
| SP3-human α-lactalbumin | MKFISILFLLIGSVFGAPVAPAEEAANHLHKRKQFTKCELSQLLKDI DGYGGIALPELICTMFHTSGYDTQAIVENNESTEYGLFQISNKLWC KSSQVPQSRNICDISCDKFLDDDITDDIMCAKKILDIKGIDYWLAHK ALCTEKLEQWLCEKL | 39 |
| SP4-human α-lactalbumin | MQFGKVLFAISALAVTALGAPVAPAEEAANHLHKRKQFTKCELSQ LLKDIDGYGGIALPELICTMFHTSGYDTQAIVENNESTEYGLFQISN KLWCKSSQVPQSRNICDISCDKFLDDDITDDIMCAKKILDIKGIDY WLAHKALCTEKLEQWLCEKL | 40 |

In some embodiments of the present invention as defined in the claims, a human α-lactalbumin is a recombinant human α-lactalbumin. In some embodiments of the present invention as defined in the claims, a recombinant human α-lactalbumin is obtained from a mammalian, fungal, yeast, bacterial, or other cell. In some embodiments of the present invention as defined in the claims, a recombinant human α-lactalbumin is not obtained from a mammalian cell. In certain embodiments of the present invention as defined in the claims, a recombinant human α-lactalbumin is obtained from a yeast cell. The yeast cell may be, for example, a *Arxula, Aspegillus, Aurantiochytrium, Candida, Claviceps, Cryptococcus, Cunninghamella, Geotrichum, Hansenula, Kluyveromyces, Kodamaea, Komagataella, Leucosporidiella, Lipomyces, Mortierella, Ogataea, Pichia, Prototheca, Rhizopus, Rhodosporidium, Rhodotorula, Saccharomyces, Schizosaccharomyces, Tremella, Trichosporon, Wickerhamomyces,* or *Yarrowia* cell. In some embodiments of the present invention as defined in the claims, the yeast cell is yeast cell is a *Komagataella* cell (e.g., *Komagataella phaffii, Komagataella pastoris, Komagataella pseudopastoris).* Additional yeast cells suitable for recombinant protein production are recognized in the art and contemplated herein. In some embodiments of the present invention as defined in the claims, a recombinant human α-lactalbumin is obtained from a bacterial cell. In other embodiments of the present invention as defined in the claims, a human α-lactalbumin is isolated from a natural source.

Embodiments of the present invention as defined in the claims include human α-lactalbumin having at least one hybrid or complex N-glycan. In some embodiments of the present invention as defined in the claims, the human α-lactalbumin comprises a glycan comprising one or more of sialic acid, galactose, N-acetylgalactosamine, or fucose. In some embodiments of the present invention as defined in the claims, the human lactoferrin comprises a bi-antennary, tri-antennary, or tetra-antennary N-glycan. Human α-lactalbumin having one or more hybrid, complex, bi-antennary, tri-antennary, or tetra-antennary N-glycan may be useful in, for example, infant formula or other nutritional compositions or supplements.

### c. Additional human milk proteins

Embodiments of the present invention as defined in the claims include as human milk proteins, but are not limited to, secretory IgA (sIgA), human serum albumin, xanthine dehydrogenase, lactoperoxidase, butyrophilin, lactadherin, adiponectin, β-casein, x-casein, leptin, osteopontin, bile salt stimulated lipase (BSSL), and lysozyme. Any one or more of these human milk proteins may be included in compositions (e.g., infant formula) .

### C. N-acetylglucosaminyltransferase

Embodiments of the present invention as defined in the claims include an N-acetylglucosaminyltransferase protein. As used herein, an "N-acetylglucosaminyltransferase protein," describes any polypeptide having N-acetylglucosaminyltransferase activity. An N-acetylglucosaminyltransferase describes an enzyme that catalyzes the transfer of a monosaccharide from specific sugar nucleotide donors onto particular hydroxyl position of a monosaccharide in a growing glycan chain in one of two possible anomeric linkages (either α or β).

An N-acetylglucosaminyltransferase protein may be an N-acetylglucosaminyltransferase protein from any suitable organism. In some embodiments of the present invention as defined in the claims, the N-acetylglucosaminyltransferase protein is a eukaryotic N-acetylglucosaminyltransferase protein. In some embodiments of the present invention as defined in the claims, the N-acetylglucosaminyltransferase protein is a mammalian N-acetylglucosaminyltransferase protein.

### 1. N-acetylglucosaminyltransferase I

In some embodiments of the present invention as defined in the claims, the N-acetylglucosaminyltransferase protein is an N-acetylglucosaminyltransferase I protein (EC 2.4.1.101). The systematic name of this enzyme class is Alpha-1,3-mannosyl-glycoprotein beta-1,2-N-acetylglucosaminyltransferase. Other names include: GnT-I, N-acetylglucosaminyltransferase I, and Uridine diphosphoacetylglucosamine-alpha-1,3-mannosylglycoprotein beta-1,2-N-acetylglucosaminyltransferase. In certain embodiments of the present invention as defined in the claims, an N-acetylglucosaminyltransferase I protein is *Homo sapiens* GnT-I, however a N-acetylglucosaminyltransferase I protein from any eukaryotic organism may be used.

### 2. β-1,2-N-acetylglucosaminyltransferase

In some embodiments of the present invention as defined in the claims, the N-acetylglucosaminyltransferase protein is a β-1,2-N-acetylglucosaminyltransferase protein (EC 2.4.1.143). The systematic name of this enzyme class is Alpha-1,6-mannosyl-glycoprotein 2-beta-N-acetylglucosaminyltransferase. Other names include: GnT-II, N-acetylglucosaminyltransferase II, and Uridine diphosphoacetylglucosamine-alpha-1,6-mannosylglycoprotein beta-1-2-N-acetylglucosaminyltransferase. In certain embodiments of the present invention as defined in the claims, a β-1,2-N-acetylglucosaminyltransferase protein is *Rattus norvegicus* GnT-II, however a β-1,2-N-acetylglucosaminyltransferase protein from any eukaryotic organism may be used.

### D. Alpha-1,3/6-Mannosidase (α-1,3/6-Mannosidase)

Embodiments of the present invention as defined in the claims include an α-1,3/6-Mannosidase protein (EC 3.2.114). As used herein, an "α-1,3/6-Mannosidase protein" (or "alpha-1,3/6-Mannosidase protein") describes any polypeptide having α-1,3/6-Mannosidase activity. An α-1,3/6-Mannosidase describes an enzyme that catalyzes removal of two mannosyl residues from N-glycans. The systematic name of this enzyme class is Mannosyl-oligosaccharide 1,3-1,6-alpha-mannosidase. Other names include: Man-II and Mannosidase II. An α-1,3/6-Mannosidase protein may be from any suitable organism. In some embodiments of the present invention as defined in the claims, the α-1,3/6-Mannosidase protein is a eukaryotic α-1,3/6-Mannosidase protein. In certain embodiments of the present invention as defined in the claims, the α-1,3/6-Mannosidase protein is *Drosophila melanogaster* Man-II, however a α-1,3/6-Mannosidase protein from any eukaryotic organism may be used.

### E. Alpha-1,2-mannosidase (α-1,2-mannosidase)

Embodiments of the present invention as defined in the claims include an α-1,2-mannosidase protein (EC 3.2.1.130). As used herein, a "α-1,2-mannosidase protein" (or "alpha-1,2-mannosidase protein") describes any polypeptide having α-1,2-mannosidase activity. The systematic name of this enzyme class is Glycoprotein endo-alpha-1,2-mannosidase. Other names include: Endo-alpha-D-mannosidase and Man-I. In some embodiments of the present invention as defined in the claims, the α-1,2-mannosidase protein is a fungal Man-I. In certain embodiments of the present invention as defined in the claims, the Man-I is a *Trichoderma reesei* Man-I.

### F. Beta-1,4- galactosyltransferase (β-1,4-galactosyltransferase)

Embodiments of the present invention as defined in the claims include a β-1,4-galactosyltransferase protein (EC 2.4.1.38). As used herein, a "β-1,4-galactosyltransferase protein" (or "beta-1,4-galactosyltransferase protein") describes any polypeptide having β-1,4-galactosyltransferase activity. The systematic name of this enzyme class is Beta-N-acetylglucosaminylglycopeptide beta-1,4-galactosyltransferase. Other names include: Glycoprotein 4-beta-galactosyltransferase, UDP-galactose--glycoprotein galactosyltransferase, and GalT. In some embodiments of the present invention as defined in the claims, the β-1,4-galactosyltransferase protein is a mammalian GalT. In certain embodiments, the GalT is a *Homo Sapiens* GalT.

### G. Glycosylated Proteins

Embodiments of the present invention as defined in the claims include glycosylated proteins (also "glycoproteins") having patterns of glycosylation similar to those of glycoproteins produced by human cells. In some embodiments of the present invention as defined in the claims, glycoproteins are N-linked glycoproteins. N-linked glycoproteins contain an N-acetylglucosamine residue linked to the amide nitrogen of an asparagine residue in the protein. The predominant sugars found on glycoproteins are glucose, galactose, mannose, fucose, N-acetylgalactosamine (GalNAc), N-acetylglucosamine (GlcNAc), and sialic acid, e.g., N-acetyl-neuraminic acid (NANA). The processing of the sugar groups occurs co-translationally in the lumen of the ER and continues in the Golgi apparatus for N-linked glycoproteins.

### H. Protein targeting

Embodiments of the present invention as defined in the claims are related to cells expressing one or more proteins from a nucleic acid molecule, where the protein is targeted to a desired subcellular location (e.g., an organelle such as the Golgi Apparatus). In some cases, a protein is targeted to a subcellular location by forming a fusion protein comprising a portion of the protein (e.g., a catalytic domain of an enzyme) and a cellular targeting signal peptide, e.g., a heterologous signal peptide (e.g., a signal peptide comprising SEQ ID NO:1, 2, 3, or 4) which is not normally ligated to or associated with the portion of the protein. The fusion protein may be encoded by a polynucleotide encoding a cellular targeting signal peptide ligated in the same translational reading frame ("in-frame") to a nucleic acid fragment encoding a protein (e.g., enzyme), or catalytically active fragment thereof.

The targeting signal peptide component of the fusion construct or protein may be derived from membrane-bound proteins of the ER or Golgi, retrieval signals, Type II membrane proteins, Type I membrane proteins, membrane spanning nucleotide sugar transporters, mannosidases, sialyltransferases, glucosidases, mannosyltransferases and phosphomannosyltransferases. In some embodiments of the present invention as defined in the claims, the targeting signal peptide is a Golgi Apparatus localization tag. Example Golgi Apparatus localization tags include, but are not limited to, a transmembrane domain from *Saccharomyces cerevisiae* Kre2p, *Saccharomyces cerevisiae* Mnn2p, *Saccharomyces cerevisiae* Mnn9, *Komagatella phaffii* Bmt2, *Komagatella phaffii* Bmt3, or *Komagatella phaffii* Ktr2.

### III. Sequences

Certain example polypeptide and nucleic sequences contemplated herein are shown below in Table 4.

**Table 4**

| **Description** | **Sequence** | **SEQ ID NO:** |
|---|---|---|
| SP1 | MKFISILFLLIGSVFGAPVNTTTEDETAQIPAEAVIGYSDLEGDFDVAVLPF SNSTNNGLLFINTTIASIAAKEEGVSLEKREAEAYVEF | 1 |
| SP2 | MQFGKVLFAISALAVTALGAPVNTTTEDETAQIPAEAVIGYSDLEGDFDV AVLPFSNSTNNGLLFINTTIASIAAKEEGVSLEKREAEAYVEF | 2 |
| SP3 | MKFISILFLLIGSVFGAPV AP AEEAANHLHKR | 3 |
| SP4 | MQFGKVLFAISALAVTALGAPVAPAEEAANHLHKR | 4 |
| SP1-hLF | MKFISILFLLIGSVFGAPVNTTTEDETAQIPAEAVIGYSDLEGDFDVAVLPF SNSTNNGLLFINTTIASIAAKEEGVSLEKREAEAYVEFGRRRSVQWCAVS QPEATKCFQWQRNMRKVRGPPVSCIKRDSPIQCIQAIAENRADAVTLDG GFIYEAGLAPYKLRPVAAEVYGTERQPRTHYYAVAVVKKGGSFQLNEL QGLKSCHTGLRRTAGWNVPIGTLRPFLNWTGPPEPIEAAVARFFSASCVP GADKGQFPNLCRLCAGTGENKCAFSSQEPYFSYSGAFKCLRDGAGDVAF IRESTVFEDLSDEAERDEYELLCPDNTRKPVDKFKDCHLARVPSHAVVA RSVNGKEDAIWNLLRQAQEKFGKDKSPKFQLFGSPSGQKDLLFKDSAIG FSRVPPRIDSGLYLGSGYFTAIQNLRKSEEEVAARRARVVWCAVGEQEL RKCNQWSGLSEGSVTCSSASTTEDCIALVLKGEADAMSLDGGYVYTAG KCGLVPVLAENYKSQQSSDPDPNCVDRPVEGYLAVAVVRRSDTSLTWN SVKGKKSCHTAVDRTAGWNIPMGLLFNQTGSCKFDEYFSQSCAPGSDPR SNLCALCIGDEQGENKCVPNSNERYYGYTGAFRCLAENAGDVAFVKDV TVLQNTDGNNNEAWAKDLKLADFALLCLDGKRKPVTEARSCHLAMAP NHAVVSRMDKVERLKQVLLHQQAKFGRNGSDCPDKFCLFQSETKNLLF NDNTECLARLHGKTTYEKYLGPQYVAGITNLKKCSTSPLLEACEFLRK | 5 |
| SP2-hLF | MQFGKVLFAISALAVTALGAPVNTTTEDETAQIPAEAVIGYSDLEGDFDV AVLPFSNSTNNGLLFINTTIASIAAKEEGVSLEKREAEAYVEFGRRRSVQ WCAVSQPEATKCFQWQRNMRKVRGPPVSCIKRDSPIQCIQAIAENRADA VTLDGGFIYEAGLAPYKLRPVAAEVYGTERQPRTHYYAVAVVKKGGSF QLNELQGLKSCHTGLRRTAGWNVPIGTLRPFLNWTGPPEPIEAAVARFFS ASCVPGADKGQFPNLCRLCAGTGENKCAFSSQEPYFSYSGAFKCLRDGA GDVAFIRESTVFEDLSDEAERDEYELLCPDNTRKPVDKFKDCHLARVPSH AVVARSVNGKEDAIWNLLRQAQEKFGKDKSPKFQLFGSPSGQKDLLFK DSAIGFSRVPPRIDSGLYLGSGYFTAIQNLRKSEEEVAARRARVVWCAVG EQELRKCNQWSGLSEGSVTCSSASTTEDCIALVLKGEADAMSLDGGYVY TAGKCGLVPVLAENYKSQQSSDPDPNCVDRPVEGYLAVAVVRRSDTSLT WNSVKGKKSCHTAVDRTAGWNIPMGLLFNQTGSCKFDEYFSQSCAPGS DPRSNLCALCIGDEQGENKCVPNSNERYYGYTGAFRCLAENAGDVAFV KDVTVLQNTDGNNNEAWAKDLKLADFALLCLDGKRKPVTEARSCHLA MAPNHAVVSRMDKVERLKQVLLHQQAKFGRNGSDCPDKFCLFQSETKN LLFNDNTECLARLHGKTTYEKYLGPQYVAGITNLKKCSTSPLLEACEFLR K | 6 |
| SP3-hLF | MKFISILFLLIGSVFGAPVAPAEEAANHLHKRGRRRSVQWCAVSQPEATK CFQWQRNMRKVRGPPVSCIKRDSPIQCIQAIAENRADAVTLDGGFIYEAG LAPYKLRPVAAEVYGTERQPRTHYYAVAVVKKGGSFQLNELQGLKSCH TGLRRTAGWNVPIGTLRPFLNWTGPPEPIEAAVARFFSASCVPGADKGQF PNLCRLCAGTGENKCAFSSQEPYFSYSGAFKCLRDGAGDVAFIRESTVFE DLSDEAERDEYELLCPDNTRKPVDKFKDCHLARVPSHAVVARSVNGKE DAIWNLLRQAQEKFGKDKSPKFQLFGSPSGQKDLLFKDSAIGFSRVPPRI DSGLYLGSGYFTAIQNLRKSEEEVAARRARVVWCAVGEQELRKCNQWS GLSEGSVTCSSASTTEDCIALVLKGEADAMSLDGGYVYTAGKCGLVPVL AENYKSQQSSDPDPNCVDRPVEGYLAVAVVRRSDTSLTWNSVKGKKSC | 7 |
| | HTAVDRTAGWNIPMGLLFNQTGSCKFDEYFSQSCAPGSDPRSNLCALCI GDEQGENKCVPNSNERYYGYTGAFRCLAENAGDVAFVKDVTVLQNTD GNNNEAWAKDLKLADFALLCLDGKRKPVTEARSCHLAMAPNHAVVSR MDKVERLKQVLLHQQAKFGRNGSDCPDKFCLFQSETKNLLFNDNTECL ARLHGKTTYEKYLGPQYVAGITNLKKCSTSPLLEACEFLRK | |
| SP4-hLF | MQFGKVLFAISALAVTALGAPVAPAEEAANHLHKRGRRRSVQWCAVSQ PEATKCFQWQRNMRKVRGPPVSCIKRDSPIQCIQAIAENRADAVTLDGGF IYEAGLAPYKLRPVAAEVYGTERQPRTHYYAVAVVKKGGSFQLNELQG LKSCHTGLRRTAGWNVPIGTLRPFLNWTGPPEPIEAAVARFFSASCVPGA DKGQFPNLCRLCAGTGENKCAFSSQEPYFSYSGAFKCLRDGAGDVAFIR ESTVFEDLSDEAERDEYELLCPDNTRKPVDKFKDCHLARVPSHAVVARS VNGKEDAIWNLLRQAQEKFGKDKSPKFQLFGSPSGQKDLLFKDSAIGFS RVPPRIDSGLYLGSGYFTAIQNLRKSEEEVAARRARVVWCAVGEQELRK CNQWSGLSEGSVTCSSASTTEDCIALVLKGEADAMSLDGGYVYTAGKC GLVPVLAENYKSQQSSDPDPNCVDRPVEGYLAVAVVRRSDTSLTWNSV KGKKSCHTAVDRTAGWNIPMGLLFNQTGSCKFDEYFSQSCAPGSDPRSN LCALCIGDEQGENKCVPNSNERYYGYTGAFRCLAENAGDVAFVKDVTV LQNTDGNNNEAWAKDLKLADFALLCLDGKRKPVTEARSCHLAMAPNH AVVSRMDKVERLKQVLLHQQAKFGRNGSDCPDKFCLFQSETKNLLFND NTECLARLHGKTTYEKYLGPQYVAGITNLKKCSTSPLLEACEFLRK | 8 |
| hLF | GRRRSVQWCAVSQPEATKCFQWQRNMRKVRGPPVSCIKRDSPIQCIQAI AENRADAVTLDGGFIYEAGLAPYKLRPVAAEVYGTERQPRTHYYAVAV VKKGGSFQLNELQGLKSCHTGLRRTAGWNVPIGTLRPFLNWTGPPEPIEA AVARFFSASCVPGADKGQFPNLCRLCAGTGENKCAFSSQEPYFSYSGAF KCLRDGAGDVAFIRESTVFEDLSDEAERDEYELLCPDNTRKPVDKFKDC HLARVPSHAVVARSVNGKEDAIWNLLRQAQEKFGKDKSPKFQLFGSPSG QKDLLFKDSAIGFSRVPPRIDSGLYLGSGYFTAIQNLRKSEEEVAARRAR VVWCAVGEQELRKCNQWSGLSEGSVTCSSASTTEDCIALVLKGEADAM SLDGGYVYTAGKCGLVPVLAENYKSQQSSDPDPNCVDRPVEGYLAVAV VRRSDTSLTWNSVKGKKSCHTAVDRTAGWNIPMGLLFNQTGSCKFDEY FSQSCAPGSDPRSNLCALCIGDEQGENKCVPNSNERYYGYTGAFRCLAE NAGDVAFVKDVTVLQNTDGNNNEAWAKDLKLADFALLCLDGKRKPVT EARSCHLAMAPNHAVVSRMDKVERLKQVLLHQQAKFGRNGSDCPDKF CLFQSETKNLLFNDNTECLARLHGKTTYEKYLGPQYVAGITNLKKCSTSP LLEACEFLRK | 9 |
| S. cerevisiae pro-MFα (1) | MRFPSIFTAVLFAASSALA | 10 |
| S. cerevisiae pro-MFα (2) | APVNTTTEDETAQIPAEAVIGYSDLEGDFDVAVLPFSNSTNNGLLFINTTI ASIAAKEEGVSLEKREAEAYVEF | 11 |
| P. pastoris Ost1 | MKFISILFLLIGSVFG | 12 |
| P. pastoris Epx1 pro region | APVAPAEEAANHLHKR | 13 |
| Pichia pastoris Pst1 | MQFGKVLFAISALAVTALG | 14 |
| gBLOCK1 | ATGCAGTTTGGAAAGGTTCTATTTGCTATTTCTGCCCTGGCTGTCACA GCTCTGGGAGCTCCAGTTGCTCCAGCCGAAGAGGCAGCAAACCACTT GCACAAGCGTATGAGGCAGGTTTGGTTCTCTTGGATTGTGGGATTGTT CCTATGTTTTTTCAACGTGTCTTCTGCTAAACGATGAAATTCATCTCA ATTCTGTTCCTTTTGATAGGCAGTGTATTTGGTATGAAATTCATCTCA ATTCTGTTCCTTTTGATAGGCAGTGTATTTGGTGCTCCAGTTGCTCCAG CCGAAGAGGCAGCAAACCACTTGCACAAGCGT | 15 |
| PMR1 | CGAAGGATCCAAACGATGAAATTCATCTCAATTC | 16 |
| PMR2 | GTAGTGTTGACTGGAGCACCAAATACAC | 17 |
| PMR3 | GGTGCTCCAGTCAACACTACAACAGAAG | 18 |
| PMR4 | TTCATCGTTTGGATCCTTCGAATAATTAGTTG | 19 |
| PMR5 | CGAAGGATCCAAACGATGCAGTTTGGAAAGG | 20 |
| PMR6 | TGACTGGAGCTCCCAGAGCTGTGACAGC | 21 |
| PMR7 | AGCTCTGGGAGCTCCAGTCAACACTACAAC | 22 |
| PMR8 | TGCATCGTTTGGATCCTTCGAATAATTAGTTGTTTTTTG | 23 |
| PMR9 | GATCCAAACGATGAAATTCATCTCAATTCTGTTCCTTTTG | 24 |
| PMR10 | TTCTTCCGGCACGCTTGTGCAAGTGGTTTG | 25 |
| PMR11 | GCACAAGCGTGCCGGAAGAAGAAGAAGTG | 26 |
| PMR12 | TGAATTTCATCGTTTGGATCCTTCGAATAATTAG | 27 |
| PMR13 | GATCCAAACGATGCAGTTTGGAAAGGTTCTATTTG | 28 |
| PMR14 | TTCTTCCGGCACGCTTGTGCAAGTGGTTTG | 29 |
| PMR15 | GCACAAGCGTGCCGGAAGAAGAAGAAGTG | 30 |
| PMR16 | CAAACTGCATCGTTTGGATCCTTCGAATAATTAG | 31 |
| PMR17 | GATCTAACATCCAAAGACGAAA | 32 |
| PMR18 | TTGAGATAAATTTCACGTTTAA | 33 |
| Full-length human lactoferrin (hLF) | MKLVFLVLLFLGALGLCLAGRRRSVQWCAVSQPEATKCFQWQRNMRK VRGPPVSCIKRDSPIQCIQAIAENRADAVTLDGGFIYEAGLAPYKLRPVAA EVYGTERQPRTHYYAVAVVKKGGSFQLNELQGLKSCHTGLRRTAGWN VPIGTLRPFLNWTGPPEPIEAAVARFFSASCVPGADKGQFPNLCRLCAGT GENKCAFSSQEPYFSYSGAFKCLRDGAGDVAFIRESTVFEDLSDEAERDE YELLCPDNTRKPVDKFKDCHLARVPSHAVVARSVNGKEDAIWNLLRQA QEKFGKDKSPKFQLFGSPSGQKDLLFKDSAIGFSRVPPRIDSGLYLGSGYF TAIQNLRKSEEEVAARRARVVWCAVGEQELRKCNQWSGLSEGSVTCSS ASTTEDCIALVLKGEADAMSLDGGYVYTAGKCGLVPVLAENYKSQQSS DPDPNCVDRPVEGYLAVAVVRRSDTSLTWNSVKGKKSCHTAVDRTAG WNIPMGLLFNQTGSCKFDEYFSQSCAPGSDPRSNLCALCIGDEQGENKC VPNSNERYYGYTGAFRCLAENAGDVAFVKDVTVLQNTDGNNNEAWAK DLKLADFALLCLDGKRKPVTEARSCHLAMAPNHAVVSRMDKVERLKQ VLLHQQAKFGRNGSDCPDKFCLFQSETKNLLFNDNTECLARLHGKTTYE KYLGPQYVAGITNLKKCSTSPLLEACEFLRK | 34 |
| Human alpha-lactalbumin (hALA) | KQFTKCELSQLLKDIDGYGGIALPELICTMFHTSGYDTQAIVENNESTEY GLFQISNKLWCKSSQVPQSRNICDISCDKFLDDDITDDIMCAKKILDIKGI DYWLAHKALCTEKLEQWLCEKL | 35 |
| Full-length hALA | MRFFVPLFLVGILFPAILAKQFTKCELSQLLKDIDGYGGIALPELICTMFHT SGYDTQAIVENNESTEYGLFQISNKLWCKSSQVPQSRNICDISCDKFLDD DITDDIMCAKKILDIKGIDYWLAHKALCTEKLEQWLCEKL | 36 |
| SP1-hALA | MKFISILFLLIGSVFGAPVNTTTEDETAQIPAEAVIGYSDLEGDFDVAVLPF SNSTNNGLLFINTTIASIAAKEEGVSLEKREAEAYVEFKQFTKCELSQLLK DIDGYGGIALPELICTMFHTSGYDTQAIVENNESTEYGLFQISNKLWCKSS QVPQSRNICDISCDKFLDDDITDDIMCAKKILDIKGIDYWLAHKALCTEK LEQWLCEKL | 37 |
| SP2-hALA | MQFGKVLFAISALAVTALGAPVNTTTEDETAQIPAEAVIGYSDLEGDFDV AVLPFSNSTNNGLLFINTTIASIAAKEEGVSLEKREAEAYVEFKQFTKCEL SQLLKDIDGYGGIALPELICTMFHTSGYDTQAIVENNESTEYGLFQISNKL WCKSSQVPQSRNICDISCDKFLDDDITDDIMCAKKILDIKGIDYWLAHKA LCTEKLEQWLCEKL | 38 |
| SP3-hALA | MKFISILFLLIGSVFGAPVAPAEEAANHLHKRKQFTKCELSQLLKDIDGYG GIALPELICTMFHTSGYDTQAIVENNESTEYGLFQISNKLWCKSSQVPQSR NICDISCDKFLDDDITDDIMCAKKILDIKGIDYWLAHKALCTEKLEQWLC EKL | 39 |
| SP4-hALA | MQFGKVLFAISALAVTALGAPVAPAEEAANHLHKRKQFTKCELSQLLKD IDGYGGIALPELICTMFHTSGYDTQAIVENNESTEYGLFQISNKLWCKSSQ VPQSRNICDISCDKFLDDDITDDIMCAKKILDIKGIDYWLAHKALCTEKLE QWLCEKL | 40 |
| SP1 (nucleic acid) | ATGAAATTCATCTCAATTCTGTTCCTTTTGATAGGCAGTGTATTTGGT GCTCCAGTCAACACTACAACAGAAGATGAAACGGCACAAATTCCGGC | 41 |
| | TGAAGCTGTCATCGGTTACTCAGATTTAGAAGGGGATTTCGATGTTGC TGTTTTGCCATTTTCCAACAGCACAAATAACGGGTTATTGTTTATAAA TACTACTATTGCCAGCATTGCTGCTAAAGAAGAAGGGGTATCTCTCG AGAAAAGAGAGGCTGAAGCTTATGTCGAGTTC | |
| SP2 (nucleic acid) | ATGCAGTTTGGAAAGGTTCTATTTGCTATTTCTGCCCTGGCTGTCACA GCTCTGGGAGCTCCAGTCAACACTACAACAGAAGATGAAACGGCACA AATTCCGGCTGAAGCTGTCATCGGTTACTCAGATTTAGAAGGGGATTT CGATGTTGCTGTTTTGCCATTTTCCAACAGCACAAATAACGGGTTATT GTTTATAAATACTACTATTGCCAGCATTGCTGCTAAAGAAGAAGGGG TATCTCTCGAGAAAAGAGAGGCTGAAGCTTATGTCGAGTTC | 42 |
| SP3 (nucleic acid) | ATGAAATTCATCTCAATTCTGTTCCTTTTGATAGGCAGTGTATTTGGT GCTCCAGTTGCTCCAGCCGAAGAGGCAGCAAACCACTTGCACAAGCG T | 43 |
| SP4 (nucleic acid) | ATGCAGTTTGGAAAGGTTCTATTTGCTATTTCTGCCCTGGCTGTCACA GCTCTGGGAGCTCCAGTTGCTCCAGCCGAAGAGGCAGCAAACCACTT GCACAAGCGT | 44 |
| Codon-optimized hLF | GCCGGAAGAAGAAGAAGTGTTCAATGGTGCGCCGTTAGTCAACCTGA GGCTACAAAGTGTTTTCAATGGCAGAGAAATATGAGAAAGGTTAGAG GTCCACCTGTTTCTTGTATCAAGAGAGATTCTCCAATCCAATGTATTC AAGCTATTGCTGAGAACAGAGCTGATGCTGTTACTTTGGATGGTGGTT TTATCTACGAAGCTGGTTTGGCTCCATATAAACTTAGACCAGTTGCTG CTGAGGTTTACGGTACTGAAAGACAACCTAGAACTCATTACTATGCT GTTGCTGTTGTTAAGAAAGGTGGTTCTTTCCAATTGAACGAATTGCAA GGTTTGAAGTCTTGTCACACTGGTTTGAGAAGAACTGCTGGTTGGAAT GTTCCAATTGGTACTTTAAGACCATTTCTTAACTGGACTGGTCCACCT GAGCCAATTGAAGCTGCTGTTGCTAGATTTTTCTCTGCTTCTTGTGTTC CAGGTGCTGATAAGGGTCAATTTCCTAATTTGTGTAGATTGTGTGCTG GTACTGGAGAGAACAAATGTGCTTTCTCTTCTCAAGAACCTTACTTTT CTTATTCTGGTGCTTTCAAGTGTTTGAGAGATGGTGCTGGAGATGTTG CTTTTATTAGAGAGTCTACTGTTTTCGAAGATTTGTCTGATGAGGCTG AAAGAGATGAGTATGAATTGTTGTGTCCAGATAACACTAGAAAGCCT GTTGATAAGTTTAAAGATTGTCATTTGGCTAGAGTTCCATCTCACGCT GTTGTTGCTAGATCTGTTAATGGTAAAGAGGATGCTATTTGGAACTTG TTGAGACAAGCTCAAGAAAAGTTCGGTAAAGACAAGTCTCCAAAGTT CCAATTGTTCGGTTCTCCTTCTGGTCAAAAGGATTTGTTGTTTAAAGA TTCTGCTATCGGTTTCTCTAGAGTTCCACCTAGAATTGATTCTGGTTTG TACTTGGGTTCTGGTTACTTCACTGCTATCCAAAATTTGAGAAAGTCT GAAGAGGAAGTTGCTGCTAGAAGAGCTAGAGTTGTTTGGTGTGCTGT TGGAGAGCAAGAATTGAGAAAGTGTAACCAATGGTCTGGTTTGTCTG AAGGTTCTGTTACTTGTTCTTCTGCTTCTACTACTGAGGATTGTATTGC TTTGGTTTTGAAAGGTGAAGCTGATGCTATGTCTTTGGATGGTGGTTA CGTTTATACTGCTGGTAAATGTGGTTTGGTTCCAGTTTTGGCTGAGAA TTACAAATCTCAACAATCTTCTGATCCAGATCCTAACTGTGTTGATAG ACCTGTTGAAGGTTATTTGGCTGTTGCTGTTGTTAGAAGATCTGATAC TTCTTTGACTTGGAACTCTGTTAAAGGTAAAAAGTCTTGTCATACTGC TGTTGATAGAACCGCCGGTTGGAATATTCCAATGGGTTTGTTGTTTAA CCAAACTGGTTCTTGTAAGTTTGATGAGTACTTCTCTCAATCTTGTGCT CCAGGTTCTGATCCTAGATCTAATTTGTGTGCTTTGTGTATTGGAGAT GAGCAAGGTGAAAACAAATGTGTTCCTAATTCTAACGAGAGATACTA TGGTTATACTGGTGCTTTTAGATGTTTGGCTGAAAACGCCGGAGATGT TGCTTTCGTTAAGGATGTTACTGTTTTGCAAAACACTGATGGTAACAA TAACGAAGCTTGGGCTAAGGATTTGAAATTGGCTGATTTCGCTTTGTT GTGTTTGGATGGTAAAAGAAAACCAGTTACTGAGGCTAGATCTTGTC ATTTGGCTATGGCTCCTAACCACGCTGTTGTTTCTAGAATGGATAAGG TTGAAAGATTGAAGCAAGTTTTGTTGCATCAACAGGCTAAGTTTGGTA GAAATGGTTCTGATTGTCCTGATAAGTTTTGTTTGTTCCAATCTGAGA CTAAAAACTTGTTGTTCAATGATAACACTGAATGTTTGGCTAGATTGC ACGGTAAAACTACTTACGAAAAATATTTGGGTCCTCAATACGTTGCTG GTATTACTAACTTGAAGAAATGCTCCACCAGTCCATTGCTTGAGGCTT GCGAGTTCCTTAGAAAATAA | 45 |
| SP1-codon optimized hLF gene | ATGAAATTCATCTCAATTCTGTTCCTTTTGATAGGCAGTGTATT TGGTGCTCCAGTCAACACTACAACAGAAGATGAAACGGCACA AATTCCGGCTGAAGCTGTCATCGGTTACTCAGATTTAGAAGGG GATTTCGATGTTGCTGTTTTGCCATTTTCCAACAGCACAAATAA CGGGTTATTGTTTATAAATACTACTATTGCCAGCATTGCTGCTA AAGAAGAAGGGGTATCTCTCGAGAAAAGAGAGGCTGAAGCTT ATGTCGAGTTCGCCGGAAGAAGAAGTGTTCAATGGTGCG CCGTTAGTCAACCTGAGGCTACAAAGTGTTTTCAATGGCAGAG AAATATGAGAAAGGTTAGAGGTCCACCTGTTTCTTGTATCAAG AGAGATTCTCCAATCCAATGTATTCAAGCTATTGCTGAGAACA GAGCTGATGCTGTTACTTTGGATGGTGGTTTTATCTACGAAGCT GGTTTGGCTCCATATAAACTTAGACCAGTTGCTGCTGAGGTTT ACGGTACTGAAAGACAACCTAGAACTCATTACTATGCTGTTGC TGTTGTTAAGAAAGGTGGTTCTTTCCAATTGAACGAATTGCAA GGTTTGAAGTCTTGTCACACTGGTTTGAGAAGAACTGCTGGTT GGAATGTTCCAATTGGTACTTTAAGACCATTTCTTAACTGGACT GGTCCACCTGAGCCAATTGAAGCTGCTGTTGCTAGATTTTTCTC TGCTTCTTGTGTTCCAGGTGCTGATAAGGGTCAATTTCCTAATT TGTGTAGATTGTGTGCTGGTACTGGAGAGAACAAATGTGCTTT CTCTTCTCAAGAACCTTACTTTTCTTATTCTGGTGCTTTCAAGT GTTTGAGAGATGGTGCTGGAGATGTTGCTTTTATTAGAGAGTC TACTGTTTTCGAAGATTTGTCTGATGAGGCTGAAAGAGATGAG TATGAATTGTTGTGTCCAGATAACACTAGAAAGCCTGTTGATA AGTTTAAAGATTGTCATTTGGCTAGAGTTCCATCTCACGCTGTT GTTGCTAGATCTGTTAATGGTAAAGAGGATGCTATTTGGAACT TGTTGAGACAAGCTCAAGAAAAGTTCGGTAAAGACAAGTCTCC AAAGTTCCAATTGTTCGGTTCTCCTTCTGGTCAAAAGGATTTGT TGTTTAAAGATTCTGCTATCGGTTTCTCTAGAGTTCCACCTAGA ATTGATTCTGGTTTGTACTTGGGTTCTGGTTACTTCACTGCTAT CCAAAATTTGAGAAAGTCTGAAGAGGAAGTTGCTGCTAGAAG AGCTAGAGTTGTTTGGTGTGCTGTTGGAGAGCAAGAATTGAGA AAGTGTAACCAATGGTCTGGTTTGTCTGAAGGTTCTGTTACTTG TTCTTCTGCTTCTACTACTGAGGATTGTATTGCTTTGGTTTTGAA AGGTGAAGCTGATGCTATGTCTTTGGATGGTGGTTACGTTTATA CTGCTGGTAAATGTGGTTTGGTTCCAGTTTTGGCTGAGAATTAC AAATCTCAACAATCTTCTGATCCAGATCCTAACTGTGTTGATA GACCTGTTGAAGGTTATTTGGCTGTTGCTGTTGTTAGAAGATCT GATACTTCTTTGACTTGGAACTCTGTTAAAGGTAAAAAGTCTTG TCATACTGCTGTTGATAGAACCGCCGGTTGGAATATTCCAATG GGTTTGTTGTTTAACCAAACTGGTTCTTGTAAGTTTGATGAGTA CTTCTCTCAATCTTGTGCTCCAGGTTCTGATCCTAGATCTAATT TGTGTGCTTTGTGTATTGGAGATGAGCAAGGTGAAAACAAATG TGTTCCTAATTCTAACGAGAGATACTATGGTTATACTGGTGCTT TTAGATGTTTGGCTGAAAACGCCGGAGATGTTGCTTTCGTTAA GGATGTTACTGTTTTGCAAAACACTGATGGTAACAATAACGAA GCTTGGGCTAAGGATTTGAAATTGGCTGATTTCGCTTTGTTGTG TTTGGATGGTAAAAGAAAACCAGTTACTGAGGCTAGATCTTGT CATTTGGCTATGGCTCCTAACCACGCTGTTGTTTCTAGAATGGA TAAGGTTGAAAGATTGAAGCAAGTTTTGTTGCATCAACAGGCT AAGTTTGGTAGAAATGGTTCTGATTGTCCTGATAAGTTTTGTTT GTTCCAATCTGAGACTAAAAACTTGTTGTTCAATGATAACACT GAATGTTTGGCTAGATTGCACGGTAAAACTACTTACGAAAAAT ATTTGGGTCCTCAATACGTTGCTGGTATTACTAACTTGAAGAA ATGCTCCACCAGTCCATTGCTTGAGGCTTGCGAGTTCCTTAGA AAATAA | 46 |
| SP2-codon optimized hLF gene | ATGCAGTTTGGAAAGGTTCTATTTGCTATTTCTGCCCTGGCTGT CACAGCTCTGGGAGCTCCAGTCAACACTACAACAGAAGATGA AACGGCACAAATTCCGGCTGAAGCTGTCATCGGTTACTCAGAT TTAGAAGGGGATTTCGATGTTGCTGTTTTGCCATTTTCCAACAG CACAAATAACGGGTTATTGTTTATAAATACTACTATTGCCAGC ATTGCTGCTAAAGAAGAAGGGGTATCTCTCGAGAAAAGAGAG GCTGAAGCTTATGTCGAGTTCGCCGGAAGAAGAAGTGTTC AATGGTGCGCCGTTAGTCAACCTGAGGCTACAAAGTGTTTTCA ATGGCAGAGAAATATGAGAAAGGTTAGAGGTCCACCTGTTTCT TGTATCAAGAGAGATTCTCCAATCCAATGTATTCAAGCTATTG CTGAGAACAGAGCTGATGCTGTTACTTTGGATGGTGGTTTTATC TACGAAGCTGGTTTGGCTCCATATAAACTTAGACCAGTTGCTG CTGAGGTTTACGGTACTGAAAGACAACCTAGAACTCATTACTA TGCTGTTGCTGTTGTTAAGAAAGGTGGTTCTTTCCAATTGAACG AATTGCAAGGTTTGAAGTCTTGTCACACTGGTTTGAGAAGAAC TGCTGGTTGGAATGTTCCAATTGGTACTTTAAGACCATTTCTTA ACTGGACTGGTCCACCTGAGCCAATTGAAGCTGCTGTTGCTAG ATTTTTCTCTGCTTCTTGTGTTCCAGGTGCTGATAAGGGTCAAT TTCCTAATTTGTGTAGATTGTGTGCTGGTACTGGAGAGAACAA ATGTGCTTTCTCTTCTCAAGAACCTTACTTTTCTTATTCTGGTGC TTTCAAGTGTTTGAGAGATGGTGCTGGAGATGTTGCTTTTATTA GAGAGTCTACTGTTTTCGAAGATTTGTCTGATGAGGCTGAAAG AGATGAGTATGAATTGTTGTGTCCAGATAACACTAGAAAGCCT GTTGATAAGTTTAAAGATTGTCATTTGGCTAGAGTTCCATCTCA CGCTGTTGTTGCTAGATCTGTTAATGGTAAAGAGGATGCTATTT GGAACTTGTTGAGACAAGCTCAAGAAAAGTTCGGTAAAGACA AGTCTCCAAAGTTCCAATTGTTCGGTTCTCCTTCTGGTCAAAAG GATTTGTTGTTTAAAGATTCTGCTATCGGTTTCTCTAGAGTTCC ACCTAGAATTGATTCTGGTTTGTACTTGGGTTCTGGTTACTTCA CTGCTATCCAAAATTTGAGAAAGTCTGAAGAGGAAGTTGCTGC TAGAAGAGCTAGAGTTGTTTGGTGTGCTGTTGGAGAGCAAGAA TTGAGAAAGTGTAACCAATGGTCTGGTTTGTCTGAAGGTTCTG TTACTTGTTCTTCTGCTTCTACTACTGAGGATTGTATTGCTTTGG TTTTGAAAGGTGAAGCTGATGCTATGTCTTTGGATGGTGGTTAC GTTTATACTGCTGGTAAATGTGGTTTGGTTCCAGTTTTGGCTGA GAATTACAAATCTCAACAATCTTCTGATCCAGATCCTAACTGT GTTGATAGACCTGTTGAAGGTTATTTGGCTGTTGCTGTTGTTAG AAGATCTGATACTTCTTTGACTTGGAACTCTGTTAAAGGTAAA AAGTCTTGTCATACTGCTGTTGATAGAACCGCCGGTTGGAATA TTCCAATGGGTTTGTTGTTTAACCAAACTGGTTCTTGTAAGTTT GATGAGTACTTCTCTCAATCTTGTGCTCCAGGTTCTGATCCTAG ATCTAATTTGTGTGCTTTGTGTATTGGAGATGAGCAAGGTGAA AACAAATGTGTTCCTAATTCTAACGAGAGATACTATGGTTATA CTGGTGCTTTTAGATGTTTGGCTGAAAACGCCGGAGATGTTGC TTTCGTTAAGGATGTTACTGTTTTGCAAAACACTGATGGTAACA ATAACGAAGCTTGGGCTAAGGATTTGAAATTGGCTGATTTCGC TTTGTTGTGTTTGGATGGTAAAAGAAAACCAGTTACTGAGGCT AGATCTTGTCATTTGGCTATGGCTCCTAACCACGCTGTTGTTTC TAGAATGGATAAGGTTGAAAGATTGAAGCAAGTTTTGTTGCAT CAACAGGCTAAGTTTGGTAGAAATGGTTCTGATTGTCCTGATA AGTTTTGTTTGTTCCAATCTGAGACTAAAAACTTGTTGTTCAAT GATAACACTGAATGTTTGGCTAGATTGCACGGTAAAACTACTT ACGAAAAATATTTGGGTCCTCAATACGTTGCTGGTATTACTAA CTTGAAGAAATGCTCCACCAGTCCATTGCTTGAGGCTTGCGAG TTCCTTAGAAAATAA | 47 |
| SP3-codon optimized hLF gene | ATGAAATTCATCTCAATTCTGTTCCTTTTGATAGGCAGTGTATT TGGTGCTCCAGTTGCTCCAGCCGAAGAGGCAGCAAACCACTTG CACAAGCGTGCCGGAAGAAGAAGTGTTCAATGGTGCGCC GTTAGTCAACCTGAGGCTACAAAGTGTTTTCAATGGCAGAGAA ATATGAGAAAGGTTAGAGGTCCACCTGTTTCTTGTATCAAGAG AGATTCTCCAATCCAATGTATTCAAGCTATTGCTGAGAACAGA GCTGATGCTGTTACTTTGGATGGTGGTTTTATCTACGAAGCTGG TTTGGCTCCATATAAACTTAGACCAGTTGCTGCTGAGGTTTACG GTACTGAAAGACAACCTAGAACTCATTACTATGCTGTTGCTGT TGTTAAGAAAGGTGGTTCTTTCCAATTGAACGAATTGCAAGGT TTGAAGTCTTGTCACACTGGTTTGAGAAGAACTGCTGGTTGGA ATGTTCCAATTGGTACTTTAAGACCATTTCTTAACTGGACTGGT CCACCTGAGCCAATTGAAGCTGCTGTTGCTAGATTTTTCTCTGC TTCTTGTGTTCCAGGTGCTGATAAGGGTCAATTTCCTAATTTGT GTAGATTGTGTGCTGGTACTGGAGAGAACAAATGTGCTTTCTC TTCTCAAGAACCTTACTTTTCTTATTCTGGTGCTTTCAAGTGTTT GAGAGATGGTGCTGGAGATGTTGCTTTTATTAGAGAGTCTACT GTTTTCGAAGATTTGTCTGATGAGGCTGAAAGAGATGAGTATG AATTGTTGTGTCCAGATAACACTAGAAAGCCTGTTGATAAGTT TAAAGATTGTCATTTGGCTAGAGTTCCATCTCACGCTGTTGTTG CTAGATCTGTTAATGGTAAAGAGGATGCTATTTGGAACTTGTT GAGACAAGCTCAAGAAAAGTTCGGTAAAGACAAGTCTCCAAA GTTCCAATTGTTCGGTTCTCCTTCTGGTCAAAAGGATTTGTTGT TTAAAGATTCTGCTATCGGTTTCTCTAGAGTTCCACCTAGAATT GATTCTGGTTTGTACTTGGGTTCTGGTTACTTCACTGCTATCCA AAATTTGAGAAAGTCTGAAGAGGAAGTTGCTGCTAGAAGAGC TAGAGTTGTTTGGTGTGCTGTTGGAGAGCAAGAATTGAGAAAG TGTAACCAATGGTCTGGTTTGTCTGAAGGTTCTGTTACTTGTTC TTCTGCTTCTACTACTGAGGATTGTATTGCTTTGGTTTTGAAAG GTGAAGCTGATGCTATGTCTTTGGATGGTGGTTACGTTTATACT GCTGGTAAATGTGGTTTGGTTCCAGTTTTGGCTGAGAATTACA AATCTCAACAATCTTCTGATCCAGATCCTAACTGTGTTGATAG ACCTGTTGAAGGTTATTTGGCTGTTGCTGTTGTTAGAAGATCTG ATACTTCTTTGACTTGGAACTCTGTTAAAGGTAAAAAGTCTTGT CATACTGCTGTTGATAGAACCGCCGGTTGGAATATTCCAATGG GTTTGTTGTTTAACCAAACTGGTTCTTGTAAGTTTGATGAGTAC TTCTCTCAATCTTGTGCTCCAGGTTCTGATCCTAGATCTAATTT GTGTGCTTTGTGTATTGGAGATGAGCAAGGTGAAAACAAATGT GTTCCTAATTCTAACGAGAGATACTATGGTTATACTGGTGCTTT TAGATGTTTGGCTGAAAACGCCGGAGATGTTGCTTTCGTTAAG GATGTTACTGTTTTGCAAAACACTGATGGTAACAATAACGAAG CTTGGGCTAAGGATTTGAAATTGGCTGATTTCGCTTTGTTGTGT TTGGATGGTAAAAGAAAACCAGTTACTGAGGCTAGATCTTGTC ATTTGGCTATGGCTCCTAACCACGCTGTTGTTTCTAGAATGGAT AAGGTTGAAAGATTGAAGCAAGTTTTGTTGCATCAACAGGCTA AGTTTGGTAGAAATGGTTCTGATTGTCCTGATAAGTTTTGTTTG TTCCAATCTGAGACTAAAAACTTGTTGTTCAATGATAACACTG AATGTTTGGCTAGATTGCACGGTAAAACTACTTACGAAAAATA TTTGGGTCCTCAATACGTTGCTGGTATTACTAACTTGAAGAAAT GCTCCACCAGTCCATTGCTTGAGGCTTGCGAGTTCCTTAGAAA ATAA | 48 |
| SP4-codon optimized hLF gene | ATGCAGTTTGGAAAGGTTCTATTTGCTATTTCTGCCCTGGCTGT CACAGCTCTGGGAGCTCCAGTTGCTCCAGCCGAAGAGGCAGCA AACCACTTGCACAAGCGTGCCGGAAGAAGAAGTGTTCAA TGGTGCGCCGTTAGTCAACCTGAGGCTACAAAGTGTTTTCAAT | 49 |
| | GGCAGAGAAATATGAGAAAGGTTAGAGGTCCACCTGTTTCTTG TATCAAGAGAGATTCTCCAATCCAATGTATTCAAGCTATTGCT GAGAACAGAGCTGATGCTGTTACTTTGGATGGTGGTTTTATCT ACGAAGCTGGTTTGGCTCCATATAAACTTAGACCAGTTGCTGC TGAGGTTTACGGTACTGAAAGACAACCTAGAACTCATTACTAT GCTGTTGCTGTTGTTAAGAAAGGTGGTTCTTTCCAATTGAACGA ATTGCAAGGTTTGAAGTCTTGTCACACTGGTTTGAGAAGAACT GCTGGTTGGAATGTTCCAATTGGTACTTTAAGACCATTTCTTAA CTGGACTGGTCCACCTGAGCCAATTGAAGCTGCTGTTGCTAGA TTTTTCTCTGCTTCTTGTGTTCCAGGTGCTGATAAGGGTCAATT TCCTAATTTGTGTAGATTGTGTGCTGGTACTGGAGAGAACAAA TGTGCTTTCTCTTCTCAAGAACCTTACTTTTCTTATTCTGGTGCT TTCAAGTGTTTGAGAGATGGTGCTGGAGATGTTGCTTTTATTAG AGAGTCTACTGTTTTCGAAGATTTGTCTGATGAGGCTGAAAGA GATGAGTATGAATTGTTGTGTCCAGATAACACTAGAAAGCCTG TTGATAAGTTTAAAGATTGTCATTTGGCTAGAGTTCCATCTCAC GCTGTTGTTGCTAGATCTGTTAATGGTAAAGAGGATGCTATTTG GAACTTGTTGAGACAAGCTCAAGAAAAGTTCGGTAAAGACAA GTCTCCAAAGTTCCAATTGTTCGGTTCTCCTTCTGGTCAAAAGG ATTTGTTGTTTAAAGATTCTGCTATCGGTTTCTCTAGAGTTCCA CCTAGAATTGATTCTGGTTTGTACTTGGGTTCTGGTTACTTCAC TGCTATCCAAAATTTGAGAAAGTCTGAAGAGGAAGTTGCTGCT AGAAGAGCTAGAGTTGTTTGGTGTGCTGTTGGAGAGCAAGAAT TGAGAAAGTGTAACCAATGGTCTGGTTTGTCTGAAGGTTCTGT TACTTGTTCTTCTGCTTCTACTACTGAGGATTGTATTGCTTTGGT TTTGAAAGGTGAAGCTGATGCTATGTCTTTGGATGGTGGTTAC GTTTATACTGCTGGTAAATGTGGTTTGGTTCCAGTTTTGGCTGA GAATTACAAATCTCAACAATCTTCTGATCCAGATCCTAACTGT GTTGATAGACCTGTTGAAGGTTATTTGGCTGTTGCTGTTGTTAG AAGATCTGATACTTCTTTGACTTGGAACTCTGTTAAAGGTAAA AAGTCTTGTCATACTGCTGTTGATAGAACCGCCGGTTGGAATA TTCCAATGGGTTTGTTGTTTAACCAAACTGGTTCTTGTAAGTTT GATGAGTACTTCTCTCAATCTTGTGCTCCAGGTTCTGATCCTAG ATCTAATTTGTGTGCTTTGTGTATTGGAGATGAGCAAGGTGAA AACAAATGTGTTCCTAATTCTAACGAGAGATACTATGGTTATA CTGGTGCTTTTAGATGTTTGGCTGAAAACGCCGGAGATGTTGC TTTCGTTAAGGATGTTACTGTTTTGCAAAACACTGATGGTAACA ATAACGAAGCTTGGGCTAAGGATTTGAAATTGGCTGATTTCGC TTTGTTGTGTTTGGATGGTAAAAGAAAACCAGTTACTGAGGCT AGATCTTGTCATTTGGCTATGGCTCCTAACCACGCTGTTGTTTC TAGAATGGATAAGGTTGAAAGATTGAAGCAAGTTTTGTTGCAT CAACAGGCTAAGTTTGGTAGAAATGGTTCTGATTGTCCTGATA AGTTTTGTTTGTTCCAATCTGAGACTAAAAACTTGTTGTTCAAT GATAACACTGAATGTTTGGCTAGATTGCACGGTAAAACTACTT ACGAAAAATATTTGGGTCCTCAATACGTTGCTGGTATTACTAA CTTGAAGAAATGCTCCACCAGTCCATTGCTTGAGGCTTGCGAG TTCCTTAGAAAATAA | |

### IV. Genetic Engineering

Vectors for transforming microorganisms (e.g., fungal cells, yeast cells) in accordance with the present invention as defined in the claims can be prepared by known techniques familiar to those skilled in the art in view of the disclosure herein. A vector typically contains one or more genes, in which each gene codes for the expression of a desired product (the gene product) and is operably linked to one or more control sequences that regulate gene expression or target the gene product to a particular location in the recombinant cell.

Exogenous nucleic acid sequences, including, for example, nucleic acid sequences encoding fusion proteins, nucleic acid sequences encoding wild-type or mutant proteins, may be introduced into many different host cells. Nucleic acid sequences configured to facilitate a genetic mutation in a gene may also be introduced into various host cells, as described further herein. Suitable host cells are microbial hosts that can be found broadly within the fungal families. Examples of suitable host strains include but are not limited to fungal or yeast species, such as *Arxula, Aspegillus, Aurantiochytrium, Candida, Claviceps, Cryptococcus, Cunninghamella, Hansenula, Kluyveromyces, Komagataella, Leucosporidiella, Lipomyces, Mortierella, Ogataea, Pichia, Prototheca, Rhizopus, Rhodosporidium, Rhodotorula, Saccharomyces, Schizosaccharomyces, Tremella, Trichosporon,* and *Yarrowia.* An exemplary host cell is a *Komagataella* cell. In some embodiments of the present invention as defined in the claims, a host cell is *Komagataella phaffii.* In some embodiments of the present invention as defined in the claims, a host cell is *Komagataella pastoris.* Another exemplary host cell is *Komagataella pseudopastoris.*

Microbial expression systems and expression vectors are well known to those skilled in the art. Any such expression vector could be used to introduce the instant genes and nucleic acid sequences into an organism. The nucleic acid sequences may be introduced into appropriate microorganisms via transformation techniques. For example, a nucleic acid sequence can be cloned in a suitable plasmid, and a parent cell can be transformed with the resulting plasmid. The plasmid is not particularly limited so long as it renders a desired nucleic acid sequence inheritable to the microorganism's progeny.

Vectors or cassettes useful for the transformation of suitable host cells are recognized in the art. Typically the vector or cassette contains a gene, sequences directing transcription and translation of a relevant gene including the promoter, a selectable marker, and sequences allowing autonomous replication or chromosomal integration. Suitable vectors comprise a region 5' of the gene harboring the promoter and other transcriptional initiation controls and a region 3' of the DNA fragment which controls transcriptional termination.

Promoters, cDNAs, and 3'UTRs, as well as other elements of the vectors, can be generated through cloning techniques using fragments isolated from native sources (Green & Sambrook, Molecular Cloning: A Laboratory Manual, (4th ed., 2012); U.S. Pat. No. 4,683,202). Alternatively, elements can be generated synthetically using known methods (Gene 164:49-53 (1995)).

### A. Vectors and Vector Components

Vectors for transforming microorganisms (e.g., yeast cells) in accordance with the present invention as defined in the claims can be prepared by known techniques familiar to those skilled in the art in view of the disclosure herein. A vector typically contains one or more genes, in which each gene codes for the expression of a desired product (the gene product) and is operably linked to one or more control sequences (e.g., promoter sequences, signal peptide sequences) that regulate gene expression or target the gene product to a particular location in the recombinant cell.

### 1. Control Sequences

Control sequences are nucleic acid sequences that regulate the expression of a coding sequence or direct a gene product to a particular location in or outside a cell. Control sequences that regulate expression include, for example, promoters that regulate transcription of a coding sequence and terminators that terminate transcription of a coding sequence. Another control sequence is a 3' untranslated sequence located at the end of a coding sequence that encodes a polyadenylation signal. Control sequences that direct gene products to particular locations include those that encode signal peptides, which direct the protein to which they are attached to a particular location inside or outside the cell.

Thus, an example vector design for expression of a gene in a microbe contains a coding sequence for a desired gene product (for example, a selectable marker, an enzyme, a fusion protein, etc.) in operable linkage with a promoter active in yeast. Alternatively, if the vector does not contain a promoter in operable linkage with the coding sequence of interest, the coding sequence can be transformed into the cells such that it becomes operably linked to an endogenous promoter at the point of vector integration. Example promoters contemplated herein include, but are not limited to, the AOX1, GAP, TEF1, TPI1, DAS1, DAS2, CAT1, and FMD promoters.

The promoter used to express a gene can be the promoter naturally linked to that gene or a different promoter.

A promoter can generally be characterized as constitutive or inducible. Constitutive promoters are generally active or function to drive expression at all times (or at certain times in the cell life cycle) at the same level. Inducible promoters, conversely, are active (or rendered inactive) or are significantly up- or down-regulated only in response to a stimulus. Both types of promoters find application in the disclosed methods. Useful inducible promoters include those that mediate transcription of an operably linked gene in response to a stimulus, such as an exogenously provided small molecule, temperature (heat or cold), lack of nitrogen in culture media, etc. Suitable promoters can activate transcription of an essentially silent gene or upregulate transcription of an operably linked gene that is transcribed at a low level.

Inclusion of termination region control sequence is optional. The termination region may be native to the transcriptional initiation region (the promoter), may be native to the DNA sequence of interest, or may be obtainable from another source (See, e.g., Chen & Orozco, Nucleic Acids Research 16:8411 (1988)).

In some cases, the full nucleotide sequence of a promoter is not necessary to drive transcription, and sequences shorter than the promoter's full nucleotide sequence can drive transcription of an operably-linked gene. The minimal portion of a promoter, termed the core promoter, includes a transcription start site, a binding site for a RNA polymerase, and a binding site for a transcription factor.

A promoter may be linked to a target by introducing the promoter and the target into a nucleic acid molecule, for example, a vector. A vector may be introduced into a cell, thereby expressing the promoter and the target. In one embodiment, a promoter is linked to a target by introducing a promoter into DNA of a cell, for example, via homologous recombination, thereby integrating the promoter into the genome of the cell.

### B. Genes and Codon Optimization

Typically, a gene includes a promoter, a coding sequence, and termination control sequences. When assembled by recombinant DNA technology, a gene may be termed an expression cassette and may be flanked by restriction sites for convenient insertion into a vector that is used to introduce the recombinant gene into a host cell. The expression cassette can be flanked by DNA sequences from the genome or other nucleic acid target to facilitate stable integration of the expression cassette into the genome by homologous recombination. Alternatively, the vector and its expression cassette may remain unintegrated (e.g., an episome), in which case, the vector typically includes an origin of replication, which is capable of providing for replication of the vector DNA.

A common gene present on a vector is a gene that codes for a protein, the expression of which allows the recombinant cell containing the protein to be differentiated from cells that do not express the protein. Such a gene, and its corresponding gene product, is called a selectable marker or selection marker. Any of a wide variety of selectable markers can be employed in a transgene construct useful for transforming the organisms covered in the present invention as defined in the claims.

For optimal expression of a recombinant protein, it may be beneficial to employ coding sequences that produce mRNA with codons optimally used by the host cell to be transformed. Thus, proper expression of transgenes can require that the codon usage of the transgene matches the specific codon bias of the organism in which the transgene is being expressed. The precise mechanisms underlying this effect are many, but include the proper balancing of available aminoacylated tRNA pools with proteins being synthesized in the cell, coupled with more efficient translation of the transgenic messenger RNA (mRNA) when this need is met. When codon usage in the transgene is not optimized, available tRNA pools may not be sufficient to allow for efficient translation of the transgenic mRNA resulting in ribosomal stalling and termination and possible instability of the transgenic mRNA.

A coding sequence can be codon optimized for a particular host cell by replacing one or more rare codons with one or more codons more frequently found in the host cell. A rare codon in a host cell describes a codon that is found in less than 5%, less than 10%, or less than 20% of coding sequences in the host cell. Rare codons can be identified using methods known to those of skill in the art.

Embodiments of the present invention as defined in the claims comprise transformation of a microorganism with a nucleic acid sequence comprising a gene that encodes a protein. The gene may be native to the cell or from a different species. The gene may be derived from a different species yet modified (e.g., codon optimized) for optimal expression in the microorganism. In certain embodiments of the present invention as defined in the claims, the gene is inheritable to the progeny of a transformed cell. In some embodiments of the present invention as defined in the claims, the gene is inheritable because it resides on a plasmid. In certain embodiments of the present invention as defined in the claims, the gene is inheritable because it is integrated into the genome of the transformed cell.

Further embodiments of the present invention as defined in the claims may comprise transformation of a microorganism with a nucleic acid sequence configured to generate a mutation in a gene of the microorganism. For example, embodiments of the present invention as defined in the claims may comprise transformation of the microorganism with a nucleic acid sequence comprising sequences upstream and downstream of a gene (e.g., an OCH1 gene), thereby facilitating reduced expression or deletion of the gene via homologous recombination. Various methods for generating mutations (including deletions or knockout mutations, as well as mutations which reduce expression of a gene) in genes of a microorganism are recognized in the art and envisioned herein. A microorganism having a deletion or knockout mutation of a gene does not product a functional copy of the protein. For example, a recombinant yeast cell of the disclosure may comprise a deletion of an endogenous OCH1 gene, such that the recombinant yeast cell does not express an endogenous, functional OCH1 protein. A microorganism having a reduced expression of a gene or protein produces a functional copy of the protein, but at a reduced amount compared with a wild-type (i.e., a non-recombinant or non-genetically modified) microorganism of the same species. Methods for reducing expression of a protein are recognized in the art and include, for example, replacement of an endogenous promoter and/or modification of one or more regulatory elements.

### C. Transformation

Cells can be transformed by any suitable technique including, e.g., biolistics, electroporation, glass bead transformation, and silicon carbide whisker transformation. Any convenient technique for introducing a transgene into a microorganism can be employed in the embodiments of the present invention as defined in the claims.

Vectors for transformation of microorganisms can be prepared by known techniques familiar to those skilled in the art. In one embodiment of the present invention as defined in the claims, an exemplary vector design for expression of a gene in a microorganism contains a gene encoding an enzyme in operable linkage with a promoter active in the microorganism. Alternatively, if the vector does not contain a promoter in operable linkage with the gene of interest, the gene can be transformed into the cells such that it becomes operably linked to a native promoter at the point of vector integration. The vector can also contain a second gene that encodes a protein. Optionally, one or both gene(s) is/are followed by a 3' untranslated sequence containing a polyadenylation signal. Expression cassettes encoding the two genes can be physically linked in the vector or on separate vectors. Co-transformation of microbes can also be used, in which distinct vector molecules are simultaneously used to transform cells (Protist 155:381-93 (2004)). The transformed cells can be optionally selected based upon the ability to grow in the presence of the antibiotic or other selectable marker under conditions in which cells lacking the resistance cassette would not grow.

### D. Genetically Engineered Cells

The present invention as defined in the claims is related to genetically engineered cells (also "engineered cells" or "recombinant cells") and methods for using such cells. In some embodiments of the present invention as defined in the claims, recombinant cells comprise one or more exogenous nucleic acid sequences. Also disclosed are methods for generating such recombinant cells comprising introducing the one or more exogenous nucleic acid sequences into a host cell. Further described are methods for collecting one or more products (e.g., a mammalian protein) from such recombinant cells comprising culturing the cells and collecting the product.

The recombinant cell may be a prokaryotic cell, such as a bacterial cell. Alternatively, the recombinant cell is a eukaryotic cell, such as a mammalian cell, a yeast cell, a filamentous fungi cell, a protist cell, an algae cell, an avian cell, a plant cell, or an insect cell. In some examples, the cell is a yeast cell. Those with skill in the art will recognize that many forms of filamentous fungi produce yeast-like growth, and the definition of yeast herein encompasses such cells. A recombinant cell of the disclosure may be selected from the group consisting of algae, bacteria, molds, fungi, plants, and yeasts. In some examples, a recombinant cell of the disclosure is a bacterial cell (e.g. *E.coli*)*,* a fungal cell, or a yeast cell.

A recombinant cell may be a recombinant fungal cell. A recombinant fungal cell may be any suitable fungal cell recognized in the art. For example, the fungal cell is an *Arxula, Aspegillus, Aurantiochytrium, Candida, Claviceps, Cryptococcus, Cunninghamella, Geotrichum, Hansenula, Kluyveromyces, Kodamaea, Komagataella, Leucosporidiella, Lipomyces, Mortierella, Ogataea, Pichia, Prototheca, Rhizopus, Rhodosporidium, Rhodotorula, Saccharomyces, Schizosaccharomyces, Tremella, Trichosporon, Wickerhamomyces,* or *Yarrowia* cell. In some embodiments of the present invention as defined in the claims, cell is *Komagataellaphaffii, Komagataella pastoris, or Pichiapastoris.*

In a particular embodiment of the present invention as defined in the claims, the yeast cell is *Kluyveromyces phaffii.*

In some embodiments of the present invention as defined in the claims, the engineered cell comprises one or more modifications for improving generation of N-glycans including human-like N-glycans. Examples of such cells and modifications are described in, for example, US patent 9,617,550.

### E. Gene Editing Systems

Gene editing techniques may be used to generate a knockout or other mutation in a gene in a population of cells. Various methods and systems for gene editing are known in the art and include, for example, zinc finger nuclease (ZFN)-based gene editing, transcription activator-like effector nuclease (TALEN)-based gene editing, and CRISPR/Cas-based gene editing. Various methods and systems for gene editing are recognized in the art and contemplated herein. Such methods may comprise CRISPR/Cas-based gene editing, which comprises the use of components of a CRISPR system, for example a guide RNA (gRNA) and a Cas nuclease. In some examples, such method does not comprise CRISPR/Cas-based gene editing (e.g., comprises ZFN-based, TALEN-based, or any other gene editing method or system).

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), and/or other sequences and transcripts from a CRISPR locus.

The CRISPR/Cas nuclease or CRISPR/Cas nuclease system can include a noncoding RNA molecule (guide) RNA, which sequence-specifically binds to DNA, and a Cas protein (e.g., Cas9), with nuclease functionality (e.g., two nuclease domains). One or more elements of a CRISPR system can derive from a type I, type II, or type III CRISPR system, e.g., derived from a particular organism comprising an endogenous CRISPR system, such as Streptococcus pyogenes.

In some examples, a Cas nuclease and gRNA (including a fusion of crRNA specific for the target sequence and fixed tracrRNA) are introduced into the cell. A Cas nuclease and a gRNA can be introduced into the cell indirectly via introduction of one or more nucleic acids (e.g., vectors) encoding for the Cas nuclease and/or the gRNA. A Cas nuclease and a gRNA can be introduced into the cell directly by introduction of a Cas nuclease protein and a gRNA molecule. In general, target sites at the 5' end of the gRNA target the Cas nuclease to the target site, e.g., the gene, using complementary base pairing. The target site may be selected based on its location immediately 5' of a protospacer adjacent motif (PAM) sequence, such as typically NGG, or NAG. In this respect, the gRNA may be targeted to the desired sequence by modifying the first 20, 19, 18, 17, 16, 15, 14, 14, 12, 11, or 10 nucleotides of the guide RNA to correspond to the target DNA sequence. In general, a CRISPR system is characterized by elements that promote the formation of a CRISPR complex at the site of a target sequence. Typically, "target sequence" generally refers to a sequence to which a guide sequence is designed to have complementarity, where hybridization between the target sequence and a guide sequence promotes the formation of a CRISPR complex. Full complementarity is not necessarily required, provided there is sufficient complementarity to cause hybridization and promote formation of a CRISPR complex.

The CRISPR system can induce double stranded breaks (DSBs) at the target site, followed by disruptions as discussed herein. In other examples, Cas9 variants, deemed "nickases," are used to nick a single strand at the target site. Paired nickases can be used, e.g., to improve specificity, each directed by a pair of different gRNAs targeting sequences such that upon introduction of the nicks simultaneously, a 5' overhang is introduced. In other examples, catalytically inactive Cas9 is fused to a heterologous effector domain such as a transcriptional repressor or activator, to affect gene expression.

The target sequence may comprise any polynucleotide, such as DNA or RNA polynucleotides. The target sequence may be located in the nucleus or cytoplasm of the cell, such as within an organelle of the cell. Generally, a sequence or template that may be used for recombination into the targeted locus comprising the target sequences is referred to as an "editing template" or "editing polynucleotide" or "editing sequence". In some examples, an exogenous template polynucleotide may be referred to as an editing template. In some examples, the recombination is homologous recombination.

Typically, in the context of an endogenous CRISPR system, formation of the CRISPR complex (comprising the guide sequence hybridized to the target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. The tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of the CRISPR complex, such as by hybridization along at least a portion of the tracr sequence to all or a portion of a tracr mate sequence that is operably linked to the guide sequence. The tracr sequence has sufficient complementarity to a tracr mate sequence to hybridize and participate in formation of the CRISPR complex, such as at least 50%, 60%, 70%, 80%, 90%, 95% or 99% sequence complementarity along the length of the tracr mate sequence when optimally aligned.

One or more vectors driving expression of one or more elements of a CRISPR system can be introduced into a cell such that expression of the elements of the CRISPR system direct formation of a CRISPR complex at one or more target sites. Components can also be delivered to cells as proteins and/or RNA. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. The vector may comprise one or more insertion sites, such as a restriction endonuclease recognition sequence (also referred to as a "cloning site"). In some examples, one or more insertion sites are located upstream and/or downstream of one or more sequence elements of one or more vectors. When multiple different guide sequences are used, a single expression construct may be used to target CRISPR activity to multiple different, corresponding target sequences within a cell.

A vector may comprise a regulatory element operably linked to an enzyme-coding sequence encoding a Cas protein (also "Cas nuclease"). Non-limiting examples of Cas proteins include Cas1, Cas1B, Cas2, Cas3, Cas4, Cas5, Cas6, Cas7, Cas8, Cas9 (also known as Csn1 and Csx12), Cas10, Cas12a (Cpf1), Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof. These enzymes are known; for example, the amino acid sequence of S. pyogenes Cas9 protein may be found in the SwissProt database under accession number Q99ZW2.

The Cas nuclease can be Cas9 (e.g., from S. pyogenes or S. pneumonia). The Cas nuclease can be Cas12a. The Cas nuclease can direct cleavage of one or both strands at the location of a target sequence, such as within the target sequence and/or within the complement of the target sequence. The vector can encode a Cas nuclease that is mutated with respect to a corresponding wild-type enzyme such that the mutated Cas nuclease lacks the ability to cleave one or both strands of a target polynucleotide containing a target sequence. In some examples, a Cas9 nickase may be used in combination with guide sequence(s), e.g., two guide sequences, which target respectively sense and antisense strands of the DNA target. This combination allows both strands to be nicked and used to induce NHEJ or HDR.

In some examples, an enzyme coding sequence encoding the CRISPR enzyme is codon optimized for expression in particular cells, such as yeast cells.

In general, a guide sequence is any polynucleotide sequence having sufficient complementarity with a target polynucleotide sequence to hybridize with the target sequence and direct sequence-specific binding of the CRISPR complex to the target sequence. In some examples, the degree of complementarity between a guide sequence and its corresponding target sequence, when optimally aligned using a suitable alignment algorithm, is or is more than 50%, 60%, 75%, 80%, 85%, 90%, 95%, 97.5%, 99%, or more.

Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), Clustal W, Clustal X, BLAST, Novoalign (Novocraft Technologies, ELAND (Illumina, San Diego, Calif.), SOAP (available at soap.genomics.org.cn), and Maq (available at maq.sourceforge.net).

The Cas nuclease may be part of a fusion protein comprising one or more heterologous protein domains. A Cas nuclease fusion protein may comprise any additional protein sequence, and optionally a linker sequence between any two domains. Examples of protein domains that may be fused to a Cas nuclease, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-5- transferase (GST), horseradish peroxidase (HRP), chloramphenicol acetyltransferase (CAT) beta galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A Cas nuclease may be fused to a gene sequence encoding a protein or a fragment of a protein that bind DNA molecules or bind other cellular molecules, including but not limited to maltose binding protein (MBP), S-tag, Lex A DNA binding domain (DBD) fusions, GAL4A DNA binding domain fusions, and herpes simplex virus (HSV) BP16 protein fusions. Additional domains that may form part of a fusion protein comprising a Cas nuclease are described in US 20110059502.

### Examples

### Example 1 - Novel signal peptides increase extracellular protein levels

To determine the effect of the novel signal peptides on extracellular protein levels, DNA encoding SEQ ID NO:1 ("SP1"), SEQ ID NO:2 ("SP2"), and SEQ ID NO:4 ("SP4") was cloned in-frame in the 5' end of the DNA coding for a protein of interest (POI), i.e., Pichia pastoris codon-optimized human lactoferrin, resulting in the substitution of the pre-pro-MFα from Saccharomyces cerevisiae. This is the most widely used signal peptide in yeast and served as the control. Single copies of the resulting sequences and the control were integrated into the AOX1 locus via double-crossover. Multiple colonies of each transformation plate were cultivated in 96-deep well plates.

To establish the presence of the protein of interest, a Western Blot was run on the supernatant. As shown in FIG. 1, when a single copy of human lactoferrin is integrated, and secretion is driven by the widely used pre-pro-MFα from Saccharomyces cerevisiae protein is not detected in the supernatant. Contrarily, extracellular protein is detected when secretion is driven by SEQ ID NO:1 ("SP1"), SEQ ID NO:2 ("SP2"), and SEQ ID NO:3 ("SP3").

With the aim of assessing the magnitude of the improvements in secretion, quantification of extracellular protein was performed via ELISA. As seen in FIG. 2, the novel engineered signals enhanced extracellular protein levels by 2.38-fold, 2.41-fold, and 2.20-fold with respect to the control (pre-pro-MFα) for SEQ ID NO:1 ("SP1"), SEQ ID NO:2 ("SP2"), and SEQ ID NO:3 ("SP3") respectively.

### Materials and Methods

Vectors and strain construction. Oligonucleotides and gBlocks were ordered from Integrated DNA Technologies (San Diego, CA, USA) and are described in Table 5. NEBuilder^{®} HiFi DNA Assembly Master Mix, OneTaq^{®} Quickload^{®} DNA polymerase, and Escherichia coli DH5α cells were from New England Biolabs. All polymerase chain reaction (PCR)-amplified sequences were confirmed via sequencing at or Genewiz.

| Table 5 | | |
|---|---|---|
| gBLOCK and Primers | | |
| SEQ ID NO: | Name | Sequence |
| 15 | gBLOCK 1 | ATGCAGTTTGGAAAGGTTCTATTTGCTATTTCTGCCCTGG CTGTCACAGCTCTGGGAGCTCCAGTTGCTCCAGCCGAAG AGGCAGCAAACCACTTGCACAAGCGTATGAGGCAGGTT TGGTTCTCTTGGATTGTGGGATTGTTCCTATGTTTTTTCA ACGTGTCTTCTGCTAAACGATGAAATTCATCTCAATTCT GTTCCTTTTGATAGGCAGTGTATTTGGTATGAAATTCATC TCAATTCTGTTCCTTTTGATAGGCAGTGTATTTGGTGCTC CAGTTGCTCCAGCCGAAGAGGCAGCAAACCACTTGCAC AAGCGT |
| 16 | PMR1 | CGAAGGATCCAAACGATGAAATTCATCTCAATTC |
| 17 | PMR2 | GTAGTGTTGACTGGAGCACCAAATACAC |
| 18 | PMR3 | GGTGCTCCAGTCAACACTACAACAGAAG |
| 19 | PMR4 | TTCATCGTTTGGATCCTTCGAATAATTAGTTG |
| 20 | PMR5 | CGAAGGATCCAAACGATGCAGTTTGGAAAGG |
| 21 | PMR6 | TGACTGGAGCTCCCAGAGCTGTGACAGC |
| 22 | PMR7 | AGCTCTGGGAGCTCCAGTCAACACTACAAC |
| 23 | PMR8 | TGCATCGTTTGGATCCTTCGAATAATTAGTTGTTTTTTG |
| 24 | PMR9 | GATCCAAACGATGAAATTCATCTCAATTCTGTTCCTTTTG |
| 25 | PMR10 | TTCTTCCGGCACGCTTGTGCAAGTGGTTTG |
| 26 | PMR11 | GCACAAGCGTGCCGGAAGAAGAAGAAGTG |
| 27 | PMR12 | TGAATTTCATCGTTTGGATCCTTCGAATAATTAG |
| 28 | PMR13 | GATCCAAACGATGCAGTTTGGAAAGGTTCTATTTG |
| 29 | PMR14 | TTCTTCCGGCACGCTTGTGCAAGTGGTTTG |
| 30 | PMR15 | GCACAAGCGTGCCGGAAGAAGAAGAAGTG |
| 31 | PMR16 | CAAACTGCATCGTTTGGATCCTTCGAATAATTAG |
| 32 | PMR17 | GATCTAACATCCAAAGACGAAA |
| 33 | PMR18 | TTGAGATAAATTTCACGTTTAA |

Transformation of linear dsDNA for integration was performed using the method described by Madden, Tolstorukov, & Cregg (2014) Fungi, Volume 1, Fungal Biology. Total yeast genomic DNA extraction was performed using the kit Easy DNA from Invitrogen (ThermoFisher, Applied Biosystems^{™}, PrepSEQ^{™} 1-2-3 Nucleic Acid Extraction Kit, Catalog number: 4452222). The resulting plasmids are summarized in Table 6.

| Table 6 | |
|---|---|
| Plasmids | |
| Name | Description |
| P1 | pPIC9 (Invitrogen) with a codon-optimized version of human lactoferrin lacking its native secretion signal. Secretion is driven by the S. cerevisiae pre-pro-MFα secretion signal. |
| P2 | P1 where the S. cerevisiae pre-pro-MFα secretion signal was substituted SEQ ID NO:1 (ostpro) |
| P3 | P1 where the S. cerevisiae pre-pro-MFα secretion signal was substituted by SEQ ID NO:2 |
| P4 | P1 where the S. cerevisiae pre-pro-MFα secretion signal was substituted by SEQ ID NO:3 |
| P5 | P1 where the S. cerevisiae pre-pro-MFα secretion signal was substituted by SEQ ID NO:4 |

The leader peptide sequences from the Pichia pastoris endogenous proteins Ost1 and Pst1 were determined using SignalP-5.0 bioinformatic software, publicly available from the Center Biological Sequence Analysis (CBS). The pro region of Epx1 was described by Heiss et al. (2015) Microbiology, 161(7).

Plasmid P1 containing the gene encoding human lactoferrin without its native secretion peptide fused in-frame with the pre-pro-leader peptide of the mating factor-alpha from Saccharomyces cerevisiae was synthesized by Genscript. The human lactoferrin gene was codon-optimized for expression in Pichia pastoris.

To create plasmid P2 containing signal sequence SP1 (SEQ ID NO:1), primers PMR1 (SEQ ID NO: 16) and PMR2 (SEQ ID NO: 17) were used to amplify the Ost1 leader sequence using gBLOCK1 as a template. The backbone containing human lactoferrin, yeast *HIS4* auxotrophic marker, and Escherichia coli antibiotic resistance and origin of replication was obtained via polymerase chain reaction (PCR) of P1 plasmid using primers PMR3 (SEQ ID NO: 18) and PMR4 (SEQ ID NO: 19). The two resulting fragments were assembled using NEBuilder^{®} HiFi DNA Assembly Master Mix following manufacturer instructions.

To generate plasmid P3 containing signal sequence SP2 (SEQ ID NO:2), primers PMR5 (SEQ ID NO:20) and PMR6 (SEQ ID NO:21) were used for amplification using gBLOCK1 (SEQ ID NO: 15) as a template. The backbone containing human lactoferrin, yeast *HIS4* auxotrophic marker, and Escherichia coli antibiotic resistance and origin of replication was obtained via PCR of P1 plasmid with primers PMR7 (SEQ ID NO:22) and PMR8 (SEQ ID NO:23). The two resulting fragments were assembled using NEBuilder^{®} HiFi DNA Assembly Master Mix following manufacturer instructions.

To generate plasmid P4 containing signal sequence SP3 (SEQ ID NO:3), primers PMR9 (SEQ ID NO:24) and PMR10 (SEQ ID NO:25) were used for amplification using the gBLOCK1 as a template. The backbone containing human lactoferrin, yeastHIS4 auxotrophic marker, and Escherichia coli antibiotic resistance and origin of replication was obtained via PCR of P1 plasmid with primers PMR11 (SEQ ID NO:26) and PMR12 (SEQ ID NO:27). The two resulting fragments were assembled using NEBuilder^{®} HiFi DNA Assembly Master Mix following manufacturer instructions.

To generate plasmid P5 containing signal sequence SP4 (SEQ ID NO:4), primers PMR13 (SEQ ID NO:28) and PMR14 (SEQ ID NO:29) were used for amplification using the gBLOCK1 (SEQ ID NO:15) as a template. The backbone containing human lactoferrin, yeast *HIS4* auxotrophic marker, and Escherichia coli antibiotic resistance and origin of replication was obtained via PCR of P1 plasmid with primers PMR15 (SEQ ID NO:30) and PMR16 (SEQ ID NO:31). The two resulting fragments were assembled using NEBuilder^{®} HiFi DNA Assembly Master Mix following manufacturer instructions.

Assembly mixtures were transformed into Escherichia coli DH5α cells as directed by the manufacturer and plated into Luria Broth (LB)-agar plates containing 100 µg/mL of ampicillin. Positive clones were selected via colony polymerase chain reaction (PCR) and inoculated overnight in 5 mL of liquid Luria Broth media supplemented with 100 µg/mL of ampicillin. Plasmids from Escherichia coli cells were isolated using GeneJET plasmid miniprep kit (ThermoFisher^{®}, Catalog number K0502). Proper assembly was confirmed via Sanger DNA sequencing.

Linear dsDNA fragment for integration into yeast was obtained using Q5 High-Fidelity DNA polymerase using primers PMR17 (SEQ ID NO:32) and PMR18 (SEQ ID NO:33) and plasmids P1, P2, P3, P4, or P5 as a template. Electrocompetent *Pichia pastoris* cells were transformed as described by Madden, Tolstorukov, & Cregg (2014) Fungi, Volume 1, Fungal Biology. Cells were spread on MD plates (1.34% yeast nitrogen base, 4×10⁻⁵ % biotin, 2% dextrose, 20% agar), which allows for selection of *his4*⁺ cells, and incubated at 30°C for seventy-two hours. Individual yeast colonies (~10-20) are then re-streaked in MD plates and allowed to grow for twenty-four hours at 30°C. Cells transformed with P1 were used as controls for assessing higher efficiency of SP1 (SEQ ID NO:1), SP2 (SEQ ID NO:2), SP3 (SEQ ID NO:3), and SP4 (SEQ ID NO:5) in the secretion of a protein of interest (POI).

Individual colonies from re-streaked plates are inoculated in 96-deep well plates using 600 µl of 2% YPD (2% dextrose, 2% peptone, 1% yeast extract). Cells were grown for forty-eight hours at 1,000 rpm and 30°C. Fifty microliters of the resulting cell suspension were transferred to 550 µl of BMG (100 mM potassium phosphate buffer (pH=6.0), 1.34% yeast nitrogen base, 4×10⁻⁵ % biotin, 1% glycerol) supplemented with 0.5% cas amino acids and incubated at 1,000 rpm and 30°C for forty-eight hours. Cells were then pelleted by centrifugation at 4,500xg for 5 minutes, and resuspended in 1% BMM (100mM potassium phosphate buffer (pH=6.0), 1.34% yeast nitrogen base, 4×10⁻⁵ % biotin, 1% methanol) for induction during seventy-two hours at 1,000 rpm and 20°C. The protein secreted to the extracellular media was then analyzed via SDS-PAGE, ELISA, and Western Blot.

### REFERENCES

The following references may provide exemplary procedural or other details supplementary to those set forth herein.
Bernauer et al., Komagataella phaffii as emerging model organism in fundamental research. Front. Microbiol. (January 11, 2021).
Besada-Lombana & Da Silva (2019) Engineering the early secretory pathway for increased protein secretion in Saccharomyces cerevisiae. Metabolic Engineering, 55, 142-151 (September 2019).
Dalvie et al. (2020) "Host-informed expression of CRISPR guide RNA for genomic engineering in Komagataella phaffii." ACS Synth. Biol., 9(1), 26-35 (December 11, 2019).
Duran & Kahve (2017) The use of lactoferrin in food industry. Academic Journal of Science, 07(02), 89-94.
Heiss et al. (2015) Multi-step processing of the secretion leader of the extracellular protein Epx1 in Pichia pastoris and implications for protein localization. Microbiology, 161(7) (July 1, 2015).
Madden, Tolstorukov, & Cregg, Book Chapter: Electroporation of Pichia pastoris. Genetic Transformation Systems 87 in Fungi, Volume 1, Fungal Biology. M.A. van den Berg and K. Maruthachalam (eds.) (2014).
Nicholl, An Introduction to Genetic Engineering. 2nd edition (Cambridge: Cambridge University Press, 2002), Glossary.
Recombinant Protein Production in Yeast, Brigitte Gasser & Diethard Mattanovich (eds.) (Springer, 2019).
U.S. Pat. No. 4,977,137 (Nicols et al.)
U.S. Pat. No. 5,571,691 (Conneely et al.)
U.S. Pat. No. 7,335,512 (Callewaert et al.)
U.S. Pat. No. 7,344,867 (Connolly)
U.S. Pat. No. 7,749,960 (Vidal et al.)
U.S. Pat. No. 7,524,815 (Vidal et al.)
U.S. Pat. No. 7,914,822 (Medo)
U.S. Pat. No. 8,440,456 (Callewaert et al.)
U.S. Pat. No. 8,871,445 (Cong et al.)
U.S. Pat. No. 8,802,650 (Buck et al.)
U.S. Pat. No. 8,821,878 (Medo et al.)
U.S. Pat. No. 8,927,027 (Fournell et al.)
U.S. Pat. No. 7,449,308 (Gerngross et al.)
U.S. Pat. Publ. 2012/0142580 (Nutten et al.)

## Claims

1. An isolated nucleic acid encoding a polypeptide comprising a sequence having at least 90% sequence identity to SEQ ID NO: 1, 2, 3, or 4.

2. The isolated nucleic acid of claim 1, wherein the polypeptide sequence comprises SEQ ID NO:1, 2, 3, or 4.

3. The isolated nucleic acid of claim 1 or 2, wherein the polypeptide further comprises a sequence of a mammalian protein.

4. The isolated nucleic acid of claim 3, wherein the mammalian protein is a human milk protein.

5. The isolated nucleic acid of claim 4, wherein the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin, optionally wherein the human milk protein is human lactoferrin.

6. The isolated nucleic acid of any one of claims 1-6, wherein the polypeptide sequence comprises SEQ ID NO:3.

7. The isolated nucleic acid of claim 6, wherein the isolated nucleic acid comprises a nucleic acid sequence having at least 80% identity to SEQ ID NO:41, 42, 43, or 44.

8. The isolated nucleic acid of claim 7, wherein the nucleic acid sequence comprises SEQ ID NO:41, 42, 43, or 44.

9. The isolated nucleic acid of claim 6, wherein the polypeptide comprises SEQ ID NO:5, 6, 7, or 8.

10. The isolated nucleic acid of claim 9, wherein the isolated nucleic acid comprises a nucleic acid sequence having at least 80% identity to SEQ ID NO:46, 47, 48, or 49.

11. The isolated nucleic acid of claim 10, wherein the nucleic acid sequence comprises SEQ ID NO:46, 47, 48, or 49.

12. A vector comprising the nucleic acid of any of claims 1-11.

13. An engineered *Pichia pastoris* cell comprising the nucleic acid of any of claims 1-11 or the vector of claim 12.

14. A method for producing a secreted protein, the method comprising growing the *Pichia pastoris* cell of claim 13 under conditions sufficient to secrete the polypeptide from the cell, optionally comprising collecting the secreted protein.

15. The method of claim 14, wherein the secreted protein is a human milk protein, optionally wherein the human milk protein is secretory IgA (sIgA), xanthine dehydrogenase, lactoferrin, lactoperoxidase, butyrophilin, lactadherin, adiponectin, β-casein, κ-casein, leptin, lysozyme, or α-lactalbumin.

16. The method of claim 14 or claim 15, wherein the human milk protein comprises one or more human-like N-glycans.

17. The method of any one of claims 14-16, further comprising generating a mixture comprising the human milk protein and one or more components of an infant formula.

18. The engineered *Pichia pastoris* cell of claim 13, wherein the nucleic acid encodes a mammalian polypeptide.

19. The engineered *Pichiapastoris* cell of claim 13, wherein the sequence comprises SEQ ID NO:1, 2, 3, or 4, optionally wherein the sequence comprises SEQ ID NO:3.

20. The engineered *Pichia pastoris* cell of claim 18, wherein the polypeptide is a mammalian protein, optionally wherein the mammalian protein is a human milk protein.

21. The engineered *Pichia pastoris* cell of claim 20, wherein the human milk protein is secretory IgA (sIgA), xanthine dehydrogenase, lactoferrin, lactoperoxidase, butyrophilin, lactadherin, adiponectin, β-casein, x-casein, leptin, lysozyme, or α-lactalbumin, optionally wherein the human milk protein is human lactoferrin.

22. The engineered *Pichia pastoris* of any one of claims 13 and 18 to 21, wherein the *Pichia pastoris* cell is *Pichia pastoris, Komagataella pastoris* or *Komagataella phaffii.*

23. The method of any one of claims 14 to 17, wherein the *Pichia pastoris* cell is *Pichia pastoris, Komagataella pastoris* or *Komagataella phaffii.*

## Patentansprüche

1. Isolierte Nukleinsäure, die für ein Polypeptid kodiert, das eine Sequenz mit mindestens 90 % Sequenzidentität zu SEQ ID NO:1, 2, 3 oder 4 umfasst.

2. Isolierte Nukleinsäure nach Anspruch 1, wobei die Polypeptidsequenz SEQ ID NO:1, 2, 3 oder 4 umfasst.

3. Isolierte Nukleinsäure nach Anspruch 1 oder 2, wobei das Polypeptid weiterhin eine Sequenz eines Säugetierproteins umfasst.

4. Isolierte Nukleinsäure nach Anspruch 3, wobei das Säugetierprotein ein menschliches Milchprotein ist.

5. Isolierte Nukleinsäure nach Anspruch 4, wobei das menschliche Milchprotein sekretorisches IgA (sIgA), Xanthindehydrogenase, Lactoferrin, Lactoperoxidase, Butyrophilin, Lactadherin, Adiponektin, β-Casein, κ-Casein, Leptin, Lysozym oder α-Lactalbumin ist, wobei optional das menschliche Milchprotein humanes Lactoferrin ist.

6. Isolierte Nukleinsäure nach einem der Ansprüche 1 bis 6, wobei die Polypeptidsequenz SEQ ID NO:3 umfasst.

7. Isolierte Nukleinsäure nach Anspruch 6, wobei die isolierte Nukleinsäure eine Nukleinsäuresequenz mit mindestens 80 % Identität zu SEQ ID NO:41, 42, 43 oder 44 umfasst.

8. Isolierte Nukleinsäure nach Anspruch 7, wobei die Nukleinsäuresequenz SEQ ID NO:41, 42, 43 oder 44 umfasst.

9. Isolierte Nukleinsäure nach Anspruch 6, wobei das Polypeptid SEQ ID NO:5, 6, 7 oder 8 umfasst.

10. Isolierte Nukleinsäure nach Anspruch 9, wobei die isolierte Nukleinsäure eine Nukleinsäuresequenz umfasst, die mindestens 80 % Identität zu SEQ ID NO:46, 47, 48 oder 49 aufweist.

11. Isolierte Nukleinsäure nach Anspruch 10, wobei die Nukleinsäuresequenz SEQ ID NO:46, 47, 48 oder 49 umfasst.

12. Vektor umfassend die Nukleinsäure nach einem der Ansprüche 1 bis 11.

13. Künstlich hergestellte *Pichia pastoris*-Zelle umfassend die Nukleinsäure nach einem der Ansprüche 1 bis 11 oder den Vektor nach Anspruch 12.

14. Verfahren zum Herstellen eines sekretierten Proteins, wobei das Verfahren umfasst Anzüchten der *Pichia pastoris*-Zelle nach Anspruch 13 unter Bedingungen, die ausreichen, um das Polypeptid aus der Zelle zu sekretieren, optional umfassend Sammeln des sezernierten Proteins.

15. Verfahren nach Anspruch 14, wobei das sekretierte Protein ein menschliches Milchprotein ist, wobei optional das menschliche Milchprotein sekretorisches IgA (sIgA), Xanthindehydrogenase, Lactoferrin, Lactoperoxidase, Butyrophilin, Lactadherin, Adiponektin, β-Casein, κ-Casein, Leptin, Lysozym oder α-Lactalbumin ist.

16. Verfahren nach Anspruch 14 oder Anspruch 15, wobei das menschliche Milchprotein ein oder mehrere menschenähnliche N-Glykane umfasst.

17. Verfahren nach einem der Ansprüche 14 bis 16, weiter umfassend Erzeugen einer Mischung umfassend das Muttermilchprotein und einen oder mehrere Bestandteile einer Säuglingsanfangsnahrung.

18. Künstlich hergestellte *Pichia pastoris*-Zelle nach Anspruch 13, wobei die Nukleinsäure für ein Säugetierpolypeptid kodiert.

19. Künstlich hergestellte *Pichia pastoris*-Zelle nach Anspruch 13, wobei die Sequenz SEQ ID NO:1, 2, 3 oder 4 umfasst, wobei optional die Sequenz SEQ ID NO:3 umfasst.

20. Künstlich hergestellte *Pichia pastoris*-Zelle nach Anspruch 18, wobei das Polypeptid ein Säugetierprotein ist, wobei optional das Säugetierprotein ein menschliches Milchprotein ist.

21. Künstlich hergestellte *Pichia pastoris*-Zelle nach Anspruch 20, wobei das menschliche Milchprotein sekretorisches IgA (sIgA), Xanthindehydrogenase, Lactoferrin, Lactoperoxidase, Butyrophilin, Lactadherin, Adiponektin, β-Casein, κ-Casein, Leptin, Lysozym oder α-Lactalbumin ist, wobei optional das menschliche Milchprotein humanes Lactoferrin ist.

22. Künstlich hergestellte Pichia pastoris nach einem der Ansprüche 13 und 18 bis 21, wobei die Pichia *pastoris*-Zelle *Pichia pastoris, Komagataella pastoris* oder *Komagataella phaffii* ist.

23. Verfahren nach einem der Ansprüche 14 bis 17, wobei die Pichia *pastoris*-Zelle Pichia pastoris, Komagataella pastoris oder Komagataella phaffii ist.

## Revendications

1. Acide nucléique isolé codant pour un polypeptide comprenant une séquence ayant au moins 90 % d'identité de séquence avec SEQ ID NO: 1, 2, 3 ou 4.

2. Acide nucléique isolé selon la revendication 1, dans lequel la séquence polypeptidique comprend SEQ ID NO: 1, 2, 3 ou 4.

3. Acide nucléique isolé selon la revendication 1 ou 2, dans lequel le polypeptide comprend en outre une séquence d'une protéine de mammifère.

4. Acide nucléique isolé selon la revendication 3, dans lequel la protéine de mammifère est une protéine de lait maternel.

5. Acide nucléique isolé selon la revendication 4, dans lequel la protéine de lait maternel est une IgA sécrétoire (sIgA), une xanthine déshydrogénase, une lactoferrine, une lactoperoxydase, une butyrophiline, une lactadhérine, une adiponectine, une β-caséine, une κ-caséine, une leptine, un lysozyme ou une α-lactalbumine, éventuellement dans lequel la protéine de lait maternel est la lactoferrine humaine.

6. Acide nucléique isolé selon l'une quelconque des revendications 1 à 6, dans lequel la séquence polypeptidique comprend SEQ ID NO: 3.

7. Acide nucléique isolé selon la revendication 6, dans lequel l'acide nucléique isolé comprend une séquence d'acide nucléique ayant au moins 80 % d'identité avec SEQ ID NO: 41, 42, 43 ou 44.

8. Acide nucléique isolé selon la revendication 7, dans lequel la séquence d'acide nucléique comprend SEQ ID NO: 41, 42, 43 ou 44.

9. Acide nucléique isolé selon la revendication 6, dans lequel le polypeptide comprend SEQ ID NO: 5, 6, 7 ou 8.

10. Acide nucléique isolé selon la revendication 9, dans lequel l'acide nucléique isolé comprend une séquence d'acide nucléique ayant au moins 80 % d'identité avec SEQ ID NO: 46, 47, 48 ou 49.

11. Acide nucléique isolé selon la revendication 10, dans lequel la séquence d'acide nucléique comprend SEQ ID NO: 46, 47, 48 ou 49.

12. Vecteur comprenant l'acide nucléique selon l'une quelconque des revendications 1 à 11.

13. Cellule de *Pichia pastoris* modifiée comprenant l'acide nucléique selon l'une quelconque des revendications 1 à 11 ou le vecteur selon la revendication 12.

14. Procédé de production d'une protéine sécrétée, le procédé comprenant la culture de la cellule de *Pichia pastoris* selon la revendication 13 dans des conditions suffisantes pour sécréter le polypeptide à partir de la cellule, comprenant éventuellement la collecte de la protéine sécrétée.

15. Procédé selon la revendication 14, dans lequel la protéine sécrétée est une protéine de lait maternel, éventuellement dans lequel la protéine de lait maternel est une IgA sécrétoire (sIgA), une xanthine déshydrogénase, une lactoferrine, une lactoperoxydase, une butyrophiline, une lactadhérine, une adiponectine, une β-caséine, une κ-caséine, leptine, lysozyme ou α-lactalbumine.

16. Procédé selon la revendication 14 ou la revendication 15, dans lequel la protéine de lait maternel comprend un ou plusieurs N-glycanes de type humain.

17. Procédé selon l'une quelconque des revendications 14 à 16, comprenant en outre la génération d'un mélange comprenant la protéine de lait maternel et un ou plusieurs composants d'une préparation pour nourrissons.

18. Cellule de *Pichia pastoris* modifiée selon la revendication 13, dans laquelle l'acide nucléique code pour un polypeptide de mammifère.

19. Cellule de *Pichia pastoris* modifiée selon la revendication 13, dans laquelle la séquence comprend SEQ ID NO: 1, 2, 3 ou 4, éventuellement dans laquelle la séquence comprend SEQ ID NO: 3.

20. Cellule de *Pichia pastoris* modifiée selon la revendication 18, dans laquelle le polypeptide est une protéine de mammifère, éventuellement dans laquelle la protéine de mammifère est une protéine de lait maternel.

21. Cellule de *Pichia pastoris* modifiée selon la revendication 20, dans laquelle la protéine de lait maternel est une IgA sécrétoire (sIgA), une xanthine déshydrogénase, une lactoferrine, une lactoperoxydase, une butyrophiline, une lactadhérine, une adiponectine, une β-caséine, une κ-caséine, une leptine, un lysozyme ou une α-lactalbumine, éventuellement dans laquelle la protéine de lait maternel est la lactoferrine humaine.

22. Pichia pastoris modifié selon l'une quelconque des revendications 13 et 18 à 21, dans lequel la cellule de *Pichia pastoris* est *Pichia pastoris, Komagataella pastoris* ou *Komagataella phaffii.*

23. Procédé selon l'une quelconque des revendications 14 à 17, dans lequel la cellule de *Pichia pastoris* est *Pichia pastoris, Komagataella pastoris* ou *Komagataella phaffii.*
